# EUROPEAN PATENT APPLICATION

(11) **EP 1 188 829 A2**
(43) Date of publication of application: **20.03.2002**
(21) Application number: 01122026.6
(22) Date of filing: 13.09.2001
(51) Int. Cl.: C12N 15/10

(54) **Antibiotic hypersusceptibility mutations in bacteria**

(30) Priority: 13.09.2000 US 232579 P; 10.09.2001 US 950319
(71) Applicant: The Board of Trustees of the University of illinois, Chicago, IL 60612 (US)
(72) Inventor: Neyfakh, Alexander A., Chicago, IL 60657-4676 (US); Vasquez-Laslop, Nora, Riverforest, IL 60305 (US)
(74) Representative: Dehmel, Albrecht, Dr.

(57) **Abstract**

The present invention discloses methods for identifying loci of antibiotic hypersusceptibility mutations using random insertional mutagenesis of a bacterial population with a selectable or screenable marker, treatment of a mutagenized bacterial population with an antibacterial agent, and selection of DNA of cells affected by the antibacterial agents. In some embodiments, the DNA selected is released from bacteria lysed in response to antibacterial treatment. The selected DNA also may be released as a result of exposure to a non-lysing antibacterial agent in combination with one or more additional treatments that results in bacterial lysis. In other instances, selected DNA may be released from bacteria only as a result of insertion of a lysis gene cassette through genetic engineering of the bacteria. In some instances, the selected DNA is used to transform fresh populations of bacteria and the cycle of DNA selection and transformation is repeated as many times as needed for obtaining hypersusceptibility mutants. After the DNA of such a mutant is collected, purified and sequenced, the location of a selectable or screenable marker identifies the antibacterial hypersusceptibility locus. The proteins encoded by these loci can serve as targets for potentiators of an antibacterial agent.

## Description

### BACKGROUND OF THE INVENTION

### 1. Field of the Invention

The present invention relates generally to the fields of bacterial resistance to antibiotics and improving susceptibility of bacteria to antibacterial agents. More particularly, it concerns methods to identify antibacterial gene loci that decrease a bacterium's susceptibility to antibacterial agents. The proteins encoded by these loci can serve as targets for potentiators of an antibacterial agent.

### 2. Description of Related Art

The looming health crisis caused by the rising bacterial resistance to antibiotics commands the need for broadening the current arsenal of antibacterial therapies. The discovery of novel antibiotics has dramatically slowed down in recent decades, suggesting that clinically useful antibacterial compounds produced in nature are limited in number and have mostly been identified.

One of the most promising approaches to improving antibacterial therapy is the combination of an antibiotic with a compound that potentiates the antibiotic's activity, such as an inhibitor of antibiotic resistance. Such inhibitors make bacterial cells more susceptible to antibiotics. Combinations of β-lactams with inhibitors of β-lactamase have been successfully used in clinics and community medicine for a number of years. Inhibitors of tetracycline-efflux transporters, the bacterial membrane proteins responsible for both intrinsic and acquired resistance to tetracycline, are in development (Levy and Nelson, 1998; Nelson and Levy, 1999).

In the early nineties, the inventors discovered the first such transporter, Bmr of *Bacillus subtilis,* and the possibility of inhibiting it with certain compounds (Neyfakh *et al.*, 1991). In the past ten years, many aspects of the biology of this and similar transporters, their regulation, and the molecular mechanism of multi drug recognition have been clarified. Since these transporters have been found to contribute significantly to the intrinsic and acquired antibiotic resistance of many pathogens, several companies are presently working on the development of transporter inhibitors that are suitable for creating two-drug combinations with antibiotics (Markham *et al.*, 1999; Renau *et al.*, 1999).

Other examples of drug combinations whose action is based on drug synergy are formulations whose individual components affect related biochemical processes within the bacterial pathogen. For example, Bactrim is a combination of sulfamethoxazole and trimethoprim, two synergistically acting compounds that inhibit subsequent steps of the bacterial folate metabolism. A more recent example is Synercid, which contains the combination of two ribosome inhibitors that together work much better than either one alone.

It is safe to assume that the examples of drug synergy listed above are only some of the multitudes of possibilities, the vast majority of which have not yet been identified. Previously identified antibiotic potentiators have been discovered either serendipitously, or on the basis of existing knowledge of bacterial drug-resistance mechanisms. The present invention offers a universal strategy for the discovery of molecular targets for potentiators of antibacterial agents. The strategy is based on an assumption that any bacterial protein whose loss-of-function mutation makes bacteria more susceptible to a particular antibacterial agent can conceivably serve as a molecular target of potentiators. Indeed, a chemical inhibitor of such a protein should mimic the effect of the mutation and potentiate the action of the antibacterial agent.

Very few hypersusceptibility mutations are known, and the majority of them affect either the permeability properties of the bacterial cell wall or antibiotic-efflux transporters.

In Gram-negative bacteria, in which the outer membrane presents a permeability barrier for many antibiotics, a number of outer membrane mutants display hypersusceptibility to various, mostly hydrophobic, antibacterial agents (Vaara, 1993). These include so-called "deep rough" mutants which produce defective lipopolysaccharide (LPS) truncated at its core oligosaccharide region, and mutants with impaired biosynthesis of lipid A, the lipid anchor of LPS (Onishi *et al*., 1996). The poor permeability of the outer membrane in Gram-negative bacteria is complemented by the activity of multidrug efflux transporters pumping antibiotics out of the bacterial cell (Markham *et al*., 1999; Renau *et al.*, 1999). Accordingly, inactivation of these transporters also leads to antibiotic hypersusceptibility (Nikaido, 1998). Similar hypersusceptible phenotypes are observed in Gram-positive bacteria upon genetic inactivation of their multidrug transporters: Bmr in *B. subtilis* (Neyfakh *et al.*, 1991; Ahmed *et al.*, 1995), NorA in *Staphylococcus aureus* (Hsieh *et al.*, 1998; Yamada *et al.*, 1997), PmrA in *Streptococcus pneumoniae* (Gill *et al.*, 1999).

It has been shown that the genetic knock-out of polynucleotide phosphorylase, an enzyme involved in RNA degradation, leads to hypersusceptibility of *E. coli* and *B*. *subtilis* to various antibiotics (McMurry and Levy, 1987; Wang and Bechhofer, 1996). In another work it was found that inactivation of protein RecG involved in recombination processes leads to an increased sensitivity of *S. aureus* to fluoroquinolones (Niga *et al*., 1997). In neither case is the hypersusceptibility particularly strong, and the molecular mechanisms of these effects have never been deciphered.

The limited number of known hypersusceptibility mutations is in sharp contrast with hundreds, if not thousands, of known mutations leading to antibiotic resistance. This disparity likely reflects the technical difficulty of identifying hypersusceptibility mutations. While it is straightforward to select for resistant bacteria that survive in the presence of an antibiotic, it is very difficult to select for those that are hypersusceptible and die in the presence of an antibiotic.

The identification of hypersusceptibility mutations requires replica plating of thousands of clones of the library of mutants in search for colonies that grow on control plates but not on plates with an antibiotic at the concentration below its minimum inhibitory concentration (MIC). Due to its extreme laboriousness, this approach has rarely been used to identify hypersusceptibility mutations. The majority of such mutations have been obtained either by genetic knockouts of known resistance genes, or simply by chance, when the hypersusceptible phenotype has been serendipitously discovered in a particular clinical isolate or a bacterial mutant originally selected for a different trait. Prior to the present invention, there has been no universal strategy for identifying molecular targets for antibiotic potentiators.

### SUMMARY OF THE INVENTION

The escalating problem of bacterial resistance to antibiotics calls for radical changes in the existing antibacterial therapies. One of the most promising approaches is the use of antibiotic potentiators, compounds that make bacterial cells hypersusceptible to broadly defined antibacterial agents, such as antibiotics, disinfectants and natural antibacterial substances of a host organism, such as molecules of specific and nonspecific immune response. The bacterial targets for potentiators can be derived from the analysis of mutations leading to hypersusceptibility to antibacterial agents. Because hypersusceptibility is a very difficult phenotype to select for, new potentiators are difficult to identify. Presently only one type of potentiator, inhibitors of β-lactamase, is widely used in medicine. Inhibitors of antibiotic-efflux transporters are under development.

The present invention discloses methods for identifying antibacterial hypersusceptibility loci. In a first embodiment, a method for identifying an antibacterial hypersusceptibility locus comprises the steps of:
(a) mutagenizing a bacterial population by random insertional mutagenesis, resulting in the insertion of a selectable and/or screenable marker in the bacterial DNA;
(b) treating the mutagenized bacterial population with an antibacterial agent or combination of agents at a concentration below that which normally affects the bacteria;
(c) collecting the DNA released into the media by the mutant bacterial cells hypersusceptible to the antibacterial treatment; and
(d) determining the location of the selectable and/or screenable marker by methods known in the art.

The location of the selectable and/or screenable marker identifies the antibacterial hypersusceptibility locus.

In some embodiments, mutagenizing comprises a transposon insertion, a prophage insertion, a transposome insertion, or an insertion by homologous recombination.

In other embodiments of the present invention, the location of the hypersusceptibility locus may be identified by transforming a second bacterial population with the DNA collected in step (c) above and selecting for integration of a selectable marker inserted into the bacterial DNA as in step (a) above. The population of transformants may or may not be treated with an antibacterial agent or combination of agents prior to determining the location of the selectable marker. Still other embodiments encompass one or more additional rounds of transformation, treatment with an antibacterial agent or combination of agents, and DNA collection prior to determination of the location of the antibacterial hypersusceptibility locus.

In some embodiments of the invention, the antibacterial agent or combination of agents may cause bacterial lysis. In such case, it may comprise a β-lactam antibiotic, including but not limited to penicillins, mecillinams, cephalosporins, clavams, 1-oxacephems, 1-carbapenems, olivanic acids, thienamycin, imipenem, nocardicins, and momobactams or, alternatively, a disinfectant. The disinfectant may be selected from an alcohol, aldehyde, anilide, biguanide, diamidine, halogen-releasing agent, silver compound, peroxygen compound, phenol, bis-phenol, halophenol and quaternary ammonium compound that causes lysis. The antibacterial agent or combination of agents may also comprise human blood or blood components causing bacterial lysis, including but not limited to plasma, serum, antibacterial antibodies, purified components of the complement system, or their combinations.

In embodiments in which the antibacterial agent or combination of agents causes bacterial lysis, DNA may be collected from the culture medium. The DNA may be collected by absorption on silica resin in the presence of guanidine, followed by elution with a water solution of low ionic strength.

In additional embodiments of the invention, the antibacterial agent or combination of agents do not cause bacterial lysis, but the bacterial populations are genetically modified to undergo lysis in response to an antibacterial agent or combination of agents. The agents may comprise an antibiotic, which includes but is not limited to a macrolide, tetracycline, ketolide, chloramphenicol, lincosamide, oxazolidinone, fluoroquinolone, rifamycin, aminoglycoside, glycopeptide, daptomycin, fusidane, sulphonamide, cycloserine, diaminopyridine, isonicotinic acid and nitrofuran. The agents may also comprise a disinfectant selected from an alcohol, aldehyde, anilide, biguanide, diamidine, halogen-releasing agent, silver compound, peroxygen compound, phenol, bis-phenol, halophenol and quaternary ammonium compound that does not cause lysis. Members of the bacterial population may contain a lysis gene cassette under the transriptional control of a promoter activated by an antibacterial agent. The lysis gene cassette may comprise a bacteriophage lysis gene, which may be inserted into the bacterial chromosome under the control of a chromosomal promoter, or may be inserted into an extrachromosomal vector.

In other embodiments, DNA may be collected following separation of bacterial cells affected by the antibacterial agent or combination of agents from bacterial cells not affected by the antibacterial agent or combination of agents. Separation of affected bacterial cells from unaffected bacterial cells may be performed on the basis of gene expression, motility, morphology, buoyant density, absorption properties, affinity for antibodies, optical properties, fluorescence properties, ability to produce fluorescent metabolites, or a combination thereof.

In some embodiments, DNA may be collected following additional treatment, wherein bacterial cells affected by the antibacterial agent or combination of agents release DNA upon the additional treatments and bacterial cells not affected by the antibacterial agent or combination of agents do not release DNA upon the additional treatments. The additional treatments include but are not limited to detergents, lysozyme, organic solvents, proteases, chaotropic agents, osmotic shock, mechanical disintegration, or a combination of these treatments.

In certain embodiments, the bacterial population may comprise *Acinetobacter spp*. and *Streptococcus spp.*

In still other embodiments, the marker of step (a) above is a screenable marker. The screenable marker may comprise a gene encoding an enzyme producing' a colored product, a gene encoding an enzyme producing a fluorescent product, or a fragment of DNA that can be detected by molecular hybridization or polymerase chain reaction.

In some embodiments the marker of step (a) is a selectable marker. In such embodiments, the selectable marker may comprise an antibiotic-resistance gene, metal-resistance gene, nutrient-utilization gene, or a gene encoding an enzyme compensating for a mutation in said bacteria.

In certain embodiments the antibacterial hypersusceptibility locus comprises a gene or operon. Additional embodiments comprise isolating a full-length copy of the gene or operon, with or without sequencing the gene or operon, with or without identifying the gene or operon from a sequenced bacterial genome.

Additional embodiments will become apparent to those of skill in the art.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
**FIG. 1 -** Inhibition of growth and induction of DNA release from the *Acinetobacter* library cells by ampicillin. Released chromosomal DNA retains high molecular weight and migrates slower than the 10 kB marker band. Two additional bands visible at the bottom of the gel in the 729 µg/ml sample appear to be rRNAs released from the cells together with DNA.
**FIG. 2 -** Changes in the amount of the released DNA in the course of selection for lysis at 3 µg/ml of ampicillin. Ten µl aliquots of the DNA samples collected over the six consecutive selection cycles were loaded on the agarose gel.
**FIG. 3 -** Identification of ampicillin-hypersusceptible clones after the SDR selection.
**FIG. 4 -** Screening of hypersusceptible colonies for the absence of the most abundant insertion of the Km^{R} cassette.
**FIG. 5 -** Characteristics of the analyzed ampicillin-hypersusceptible mutants of *Acinetobacter sp.*
**FIG. 6 -** Gene organization in the *alkM* model strain and induction of DNA release before and after SDR selection. Transformants 1 and 2 and the derivative of the transformant 1 after four cycles of SDR selection were grown to OD₆₀₀ 0.3 and a drop of hexadecane was added to 5 ml of the culture. After overnight incubation (induction of the *alkM* expression occurs only in stationary phase (Ratajczak *et al.*, *J. Bacteriol*. 180(22):5822-7, 1998)) the released DNA was purified from the medium as described in the text.
**FIG. 7** - Effects of chloramphenicol, erythromycin and tetracycline on the release of DNA and growth of the LIT1 strain.
**FIG. 8 -** The scheme of inverse PCR used to identify the site of insertion of the lysis cassette in the LIT1 strain. Small arrows indicate PCR primers.

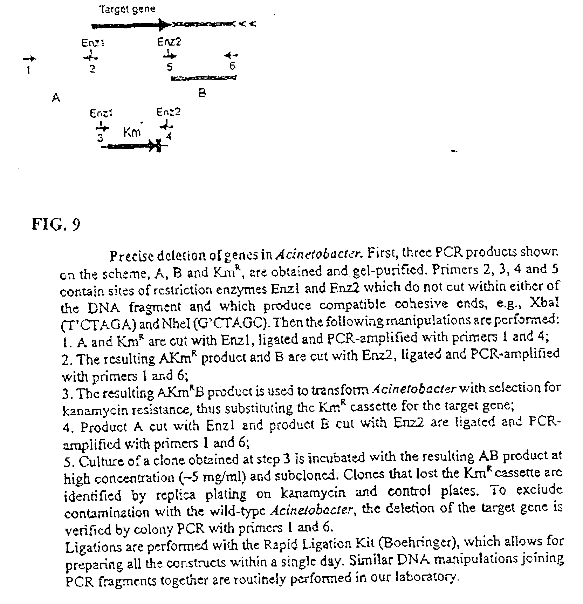

**FIG. 9 -** Precise deletion of genes in *Acinetobacter.* A, B and Km^{R} are PCR products. Primers 2, 3, 4 and 5 contain sites of restriction enzymes Enz1 and Enz2 which do not cut within either of the DNA fragments and which produce compatible cohesive ends. The AKm^{R}B product obtained was used to transform *Acinetobacter* with selection for kanamycin resistance, thus substituting the Km^{R} cassette for the target gene. Culture of a clone containing the Km^{R} cassette was incubated with the AB product and subcloned. Clones that lost the Km^{R} cassette were identified by replica plating.

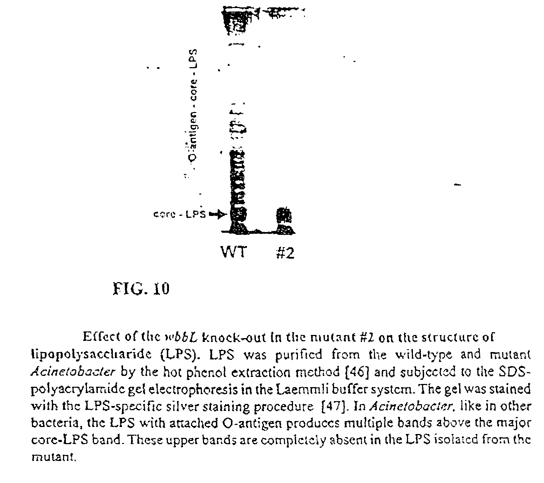

**FIG. 10 -** Effect of the *wbbL* knock-out in the mutant #2 on the structure of lipopolysaccharide (LPS). LPS was purified from the wild-type and mutant *Acinetobacter* by the hot phenol extraction method (Westphal and Jann, 1965) and subjected to the SDS-polyacrylamide gel electrophoresis in the Laemmli buffer system. The gel was stained with the LPS-specific silver staining procedure (Tsai and Frasch, 1982). In *Acinetobacter*, like in other bacteria, the LPS with attached O-antigen produces multiple bands above the major core-LPS band. These upper bands are completely absent in the LPS isolated from the mutant.

### DETAILED DESCRIPTION

In accordance with the present invention there may be employed conventional molecular biology, microbiology, and recombinant DNA techniques within the skill of the art. Such techniques are explained fully in the literature. See, *e.g.,* Sambrook, Fritsch and Maniatis, "Molecular Cloning: A Laboratory Manual," Second Edition (1989) Cold Spring Harbor Laboratory Press, Cold Spring Harbor, N.Y. (herein "Sambrook *et al.*, 1989"); "DNA Cloning: A Practical Approach," Volumes I and II (D. N. Glover ed. 1985). Each of these references is specifically incorporated herein by reference.

### The Present Invention

The inventors now provide a novel method for identifying multiple antibiotic hypersusceptibility mutations using a novel selection strategy. Unlike the traditional methods of selection, the present invention involves selection not of surviving or growing cells, but of DNA released from dying bacteria. More specifically, a library of bacteria carrying random insertions of a marker gene in their chromosomes is treated with an antibiotic at below minimum inhibitory concentrations (MIC) and the DNA that is released into the medium is collected. A fresh batch of bacteria is then transformed with the released DNA and selected for a genetic marker. The marker gene integrates into the chromosome of the recipient bacteria and generates the same mutation. The obtained secondary library is subjected to the same treatment as the original library and the cycle is repeated as many times as needed.

The insertions that lead to antibiotic hypersusceptibility undergo enrichment in each round of selection. Five cycles of selection result in the display of the hypersusceptibility phenotype by the majority of clones in a library. Since the entire procedure selects for co-segregation of this phenotype with the marker gene, the hypersusceptibility is linked to the insertion of the marker into a particular genetic locus. The site of insertion in each mutant clone is then determined by direct sequencing, thus revealing the gene for a potential target of an antibiotic potentiator. This method is referred to in this disclosure as the SDR (selection for DNA release) method.

The disclosed method is applicable to antibiotics, such as most β-lactams, that cause cell lysis and the release of bacterial DNA. β-lactam antibiotics comprise approximately half of the current antibiotic usage. The creation of reporter strains of bacteria that lyse in response to antibiotics that normally do not cause bacterial lysis permits extension of the invention to other antibacterial agents. In these strains, the lysis genes of bacteriophage λ are placed under control of the bacterial promoters activated by antibiotics. A strain that lyses in the presence of translational inhibitors (chloramphenicol, tetracycline, and erythromycin) is disclosed. With these features, the disclosed SDR method is applicable to many antibiotics, possibly all of them.

Bacteria can become hypersusceptible to antibacterial agents by mutation of the bacterial chromosome. Identification and selection of hypersusceptible mutant bacteria permits identification of the sites of mutations in the bacterial chromosome. The gene or operon encoding resistance or decreased susceptibility to an antibacterial agent may be isolated and sequenced using methods well known in the art. The location of the mutations specifies bacterial proteins whose inhibitors may potentiate the action of an antibacterial agent.

A number of other medically-oriented applications of the SDR method are envisioned. For example, the selection of bacterial mutants hypersusceptible to human serum is envisioned. Such selection provides information for developing a novel class of antibacterial drugs, whose action is based entirely on synergy with the protective mechanisms of human organism.

### Definitions

Therefore, if appearing herein, the following terms shall have the definitions set out below.

A cell has been "transformed" by exogenous or heterologous DNA when such DNA has been introduced inside the cell. The transforming DNA may or may not be integrated (covalently linked) into chromosomal DNA making up the genome of the cell.

The term "hypersusceptible bacterium" or "hypersusceptible strain" is used in describing the effect of an antibacterial agent. This terms means that the minimum inhibitory concentration (MIC) of a specific antibacterial agent for such a bacterium is lower than that for an original bacterium or strain.

A "potentiator" or "potentiating compound" refers to a compound which has a synergistic, or greater than additive, effect on antibacterial activity when used with an antibacterial agent. Thus, a potentiator enhances the antibacterial effect of an antibacterial agent when the two compounds are used in combination, but does not have significant antibacterial activity when used alone at concentrations similar to its concentration in the combination use. For evaluating the intrinsic antibacterial activity of a possible potentiator, the reduction in growth of a bacterium in the presence of a possible potentiator is determined in comparison to the growth of the bacterium in the absence of the possible potentiator.

The site of a mutation in the bacterial chromosome that provides the bacterium's hypersusceptibility to antibacterial agents is termed an "antibacterial hypersusceptibility locus." This locus is the location on a chromosome of a gene or operon encoding resistance or decreased susceptibility to an antibacterial agent. The locus may comprise a single gene or an operon. The locus is identified by the method disclosed in the present application.

### Target Bacteria

Any bacteria that efficiently take up DNA and possess an effective system of homologous recombination can be employed with the SDR method. One such bacterial species is Gram-negative bacterium *Acinetobacter sp.* strain BD413 (also known as ADP1), which demonstrates a phenomenal transformation efficiency (Juni and Janik, 1969; Palmen *et al.*, 1993). *Acinetobacter* species, especially *A. baumanii*, are becoming an increasingly serious medical problem, especially in the hospital setting. Currently, about 1.5% of nosocomial infections is caused by this opportunistic pathogen and this number is rapidly rising, especially in Europe and Southeast Asia. *Acinetobacter* infections are intrinsically resistant to a number of antibiotics and cause high mortality (Bergogne-Berezin and Towner, 1996), thus demanding an intensive search for new therapies. Moreover, the clinical significance of the present invention extends beyond *Acinetobacter* infections because the majority of the potentiator targets identified in this organism are also likely to be relevant in other Gram-negative bacterial pathogens, such as *Pseudomonas* and *Salmonella*, and possibly all bacteria.

*Acinetobacter sp.* belongs to the group Moraxellaceae, which was originally classified as *Bacterium anitratum*, later as *Acinetobacter calcoaceticus* and finally simply as *Acinetobacter* sp. The strain BD413 has remarkable genetic competence (Juni and Janik, 1969). Approximately 10% of cells of *Acinetobacter* sp. can be transformed simply by adding 1-2 µg of a fragment of chromosomal DNA with inserted antibiotic-resistance marker to one ml of the exponentially growing culture of *Acinetobacter* (Palmen *et al*., 1993). The lack of pathogenicity of strain BD413 is beneficial to its use in genetics experimentation.

The extremely high transformation efficiency of *Acinetobacter* and the clinical importance of many closely related *Acinetobacter* species favored the choice of this organism as a representative Gram-negative species in the studies.

Although *Acinetobacter* is disclosed in the present invention, the SDR method can also be employed with Gram-positive cocci *Streptococcus pneumoniae*. The rate of transformation of this bacterium also is very high and can be further increased by the natural competency-inducing peptide (Morrison, 1997).

Members of the bacterial population may contain an artificially inserted lysis gene cassette under the transriptional control of a promoter activated by an antibacterial agent or combination of agents. The lysis gene cassette may comprise a bacteriophage lysis gene, or a combination of genes, which may be inserted into the bacterial chromosome under the control of a chromosomal promoter, or may be inserted into an extrachromosomal vector.

### Markers

Cells containing a mutation of interest in the present invention may'be identified *in vitro* or *in vivo* by including a marker in the transformed cell. Such markers would confer an identifiable change to the cell permitting easy identification of cells containing the potential mutation. Generally, a selectable marker is one that confers a property that allows for selection. An example of a selectable marker is a drug resistance marker, such as an antibiotic-resistance marker. Also contemplated are selectable markers that encode an enzyme that compensates for a mutation of interest, markers that confer metal resistance, and markers that confer nutrient utilization.

In addition to markers conferring a phenotype that allows for the discrimination of transformants based on the implementation of conditions, other types of markers including screenable markers also are contemplated. For example, markers comprising a gene encoding an enzyme that produces a colored product or a fluorescent product are contemplated, as are DNA fragments that can be detected by molecular hybridization or polymerase chain reaction. One of skill in the art would also know how to employ immunologic markers, possibly in conjunction with FACS analysis.

### Suitable Antibacterial Agents

Numerous antibacterial agents or combinations of agents are suitable for use in the present invention. Included are agents that cause bacterial lysis, such as β-lactam antibiotics, disinfectants, and human blood or blood components. Suitable β-lactam antibiotics include, but are not limited to penicillins, mecillinams, cephalosporins, clavams, 1-oxacephems, 1-carbapenems, olivanic acids, thienamycin, imipenem, nocardicins, and momobactams. Suitable disinfectants may be chosen from an alcohol, aldehyde, anilide, biguanide, diamidine, halogen-releasing agent, silver compound, peroxygen compound, phenol, bis-phenol, halophenol and quaternary ammonium compound. Suitable human blood components include plasma, serum, antibacterial antibodies, and purified components of the complement system.

Antibacterial agents that do not normally cause bacterial lysis also can be employed in the present invention. In such case, bacteria can be genetically modified to undergo lysis in response to an antibacterial agent or combination of agents. These agents include antibiotics and disinfectants. Suitable antibiotics include, but are not limited to a macrolide, tetracycline, ketolide, chloramphenicol, lincosamide, oxazolidinone, fluoroquinolone, rifamycin, aminoglycoside, glycopeptide, daptomycin, fusidane, sulphonamide cycloserine, diaminopyridine, isonicotinic acid, nitrofuran. Suitable disinfectants may be chosen from an alcohol, aldehyde, anilide, biguanide, diamidine, halogen-releasing agent, silver compound, peroxygen compound, phenol, bis-phenol, halophenol and quaternary ammonium compound.

The use of bacteriostatic antibacterial agents or combination of agents that do not cause lysis of bacteria can be employed in the present invention. Use of bacteriostatic antibacterial agents, in combination with additional treatment of the bacteria, can result in lysis of bacterial cells affected by the antibacterial agent or combination of agents. Such bacteriostatic antibacterial agents include, but are not limited to a macrolide, tetracycline, ketolide, chloramphenicol, lincosamide, oxazolidinone, fluoroquinolone, rifamycin, aminoglycoside, glycopeptide, daptomycin, fusidane, sulphonamide cycloserine, diaminopyridine, isonicotinic acid or nitrofuran. The additional treatments causing release of the bacterial DNA include, but are not limited to detergents, lysozyme, organic solvents, proteases, chaotropic agents, osmotic shock, mechanical disintegration, or a combination of these treatments.

### Mutagenesis

The method of the present invention involves mutagenesis of bacteria to create a library of bacteria mutagenized by random insertion of a marker gene into the bacterial chromosome. Insertion mutagenesis involves the induction of mutation by the insertion of another piece of DNA into the target gene. Because it involves the insertion of some type of DNA fragment, the mutations generated are generally loss-of-function, rather than gain-of-function mutations.

Mutagenesis may include, but is not limited to, transposon insertion, prophage insertion, transposome insertion, and insertion by homologous recombination. Each of these methods of mutagenesis is well known in the art.

Transposable genetic elements are DNA sequences that can move (transpose) from one place to another in the genome of a cell. The first transposable elements to be recognized were the Activator/Dissociation elements of *Zea mays* (McClintock, 1957). Since then, they have been identified in a wide range of organisms, both prokaryotic and eukaryotic.

Most transposable elements have inverted repeat sequences at their termini. These terminal inverted repeats may be anything from a few bases to a few hundred bases long and in many cases they are known to be necessary for transposition. Prokaryotic transposable elements have been most studied in *E. coli* and Gram negative bacteria, but also are present in Gram positive bacteria. They are generally termed insertion sequences if they are less than about 2 kB long, or transposons if they are longer. Elements of each type encode at least one polypeptide, a transposase, required for their own transposition. Transposons often further include genes coding for function unrelated to transposition, for example, antibiotic resistance genes.

Artificial transposon mutagenesis in bacteria with the purpose of creating a library of insertion mutants is usually carried out by introducing into bacteria a specially constructed plasmid containing a selectable or screenable marker gene surrounded by inverted repeats and the gene of a transposase located either between the repeats or elsewhere in the plasmid. The region of DNA between the repeats is transposed, under the action of transposase, into the bacterial chromosome. The plasmid itself is then removed, usually by transferring the bacterial population into the environment preventing plasmid replication, for example, a high temperature environment.

Mutagenesis via transposome insertion was first described by Goryshin *et al*. in 2000, and involves the use of a purified transposase with an artificial DNA construct containing a selectable antibiotic-resistance marker surrounded by inverted repeats. The complex, introduced into bacterial population by electrotransformation, randomly integrates into the bacterial chromosome. The bacteria with integrated transposomes are then selected by applying antibiotic to which the gene located within the transposome provides resistance.

A bacteriophage genome integrated into the chromosome of its bacterial host cell is a prophage. Phages that can integrate into the host genome are known as temperate phages, and cells carrying such prophages are known as lysogens. Prophages can remain in the chromosome for many cell generations until some stimulus leads to their induction. The phage genome then excises from the chromosome and enters the lytic cycle of phage replication. A number of temperate phages, such as mu or its modified variants, can be used to create a library of insertion mutants.

Mutagenesis also may be accomplished by homologous recombination. Homologous recombination is a reaction between any pair of DNA sequences having a similar sequence of nucleotides (homologous sequences), where the two sequences interact (recombine) to form a new recombinant DNA species. The frequency of homologous recombination increases as the length of the shared nucleotide DNA sequences increases. Homologous recombination can occur between two DNA sequences that are less than identical, but the recombination frequency declines as the divergence between the two sequences increases.

To generate a library of mutants by homologous recombination the randomly fragmented bacterial DNA is ligated, with the help of DNA-ligase, with a selectable marker gene. The formed products of the ligation are then introduced into bacteria where they recombine wit the chromosomal DNA leading to the insertion of the marker gene into the chromosome.

### Selection of Mutation Libraries

The mutation libraries of the present invention comprise a collection of bacterial mutants generated by random insertional mutagenesis resulting in the insertion of a selectable or screenable marker into the DNA of a bacterial population.

One novel aspect of the present invention involves the selection, not of surviving or growing bacterial cells, but of DNA released from dying bacteria. More specifically, a library of bacteria as described above, carrying random insertions of a marker gene in their chromosomes, is treated with an antibacterial agent at a concentration lower than that normally affecting such bacteria, for instance, a concentration below the minimum inhibitory concentration (MIC). The DNA that is released into the medium is collected. A fresh batch of bacteria is then transformed with the released DNA and selected for a genetic marker. The marker gene integrates into the chromosome of the recipient bacteria by homologous recombination of the flanking regions of DNA. The secondary library thus obtained is subjected to the same treatment as the original library and the cycle is repeated as many times as needed.

The insertions that lead to antibiotic hypersusceptibility undergo enrichment in each round of selection. The inventors have found that five cycles of selection result in the display of the hypersusceptibility phenotype by the majority of clones in a library. Since the entire procedure selects for co-segregation of the hypersusceptible phenotype with the marker gene, the hypersusceptibility is linked to the insertion of the marker into a particular genetic locus. The site of insertion in each mutant clone is then determined by sequencing, thus revealing the gene for a potential target of an antibiotic potentiator. Any method of DNA sequencing known in the art may be employed in the present invention.

The method described above is applicable to antibiotics, such as most β-lactams, that cause cell lysis and the release of bacterial DNA. β-lactam antibiotics comprise approximately half of the current antibiotic usage. The method also may be used with antibiotics that do not cause cell lysis of wild type bacteria, but wherein the bacteria lyse upon additional treatment. The method may be used with reporter strains of bacteria that are genetically engineered to lyse in response to antibiotics that do not normally cause bacterial cell lysis. With these features, the disclosed SDR method is applicable to many antibacterial agents, possibly all of them.

### Generation of Acinetobacter libraries comprising a kanamycin resistance cassette

In the present invention a library of mutants was generated by ligating random fragments of the chromosomal DNA of *Acinetobacter* with a kanamycin resistance cassette (KM^{R} ) and then transforming *Acinetobacter* with the ligation mixture. A similar approach was used by Palmen *et al*. to select for transformation-deficient mutants of *Acinetobacter* (Palmen and Hellingwerf, 1997; Palmen *et al.*, 1992). The 950 bp KM^{R} cassette was constructed by attaching a constitutive *trc* promoter upstream of the *nptI* (neomycin phosphotransferase) gene which was PCR-amplified from the commercially available pET30 EK/Lic vector (Novagen).

Fragments of the genome were obtained by completely digesting *Acinetobacter* chromosomal DNA with frequently cutting restriction enzymes: TaqI (T'CGA), Sau3A1 ('GATC) and a mixture of HpaII (C'CGG) and Hin6I (G'CGC). Each digestion produced a set of DNA fragments with the median length of approx. 300 bp. The Km^{R} cassette was amplified by PCR with primers containing restriction sites for Bsp 119I (TT'CGAA; compatible with HpaII, Hin6I and TaqI) and BcII (T'GATCA; compatible with Sau3A1). After digestion of the amplified KM^{R} gene with either Bsp 119I or BclI, it was ligated with the compatibly digested *Acinetobacter* DNA. The ligation mixtures were separately added to 1 ml of the *Acinetobacter* culture in LB medium (OD₆₀₀ 0.3) and, after an hour, cells were plated on LB plates with kanamycin (25 µg/ml). The resulting libraries contained 7,500 (HpaII+Hin6I), 2,500 (TaqI), and 1,200 (Sau3A1) kanamycin-resistant clones. All three libraries were pooled together to create a library of ~ 11,000 clones containing KM^{R} insertions.

In a separate instance, a 28,000 member library of mutants was generated by the use of KM^{R} gene incorporated into a transposome according to the technique described in Goryshin *et al*.

The disclosed method can be employed in the generation of libraries of other Gram-negative and of Gram-positive bacteria comprising a bacterial resistance cassette.

### Separation of bacterial cells affected by antibacterial agent(s) from those not affected

In some embodiments of the present invention, bacterial cells affected by an antibacterial agent or combination of agents are separated from bacterial cells not so affected, prior to collecting DNA from affected bacterial cells. This separation can be performed on the basis of gene expression, motility, morphology, buoyant density, absorption properties, affinity for antibodies, optical properties, fluorescence properties, ability to produce fluorescent metabolites, or a combination thereof. Any method for the above techniques for separation of affected and unaffected cells known to those of skill in the art can be employed in the present invention.

For example, *Acinetobacter* treated with human serum releases only small amounts of DNA, but cells affected by serum changed in both size and buoyant density. Such affected cells were successfully separated form unaffected cells by centrifugation, with affected cells sedimenting at low rate. DNA was then isolated form the affected cells.

### Collecting DNA from mutant libraries and selection of the library clones hypersusceptible to ampicillin

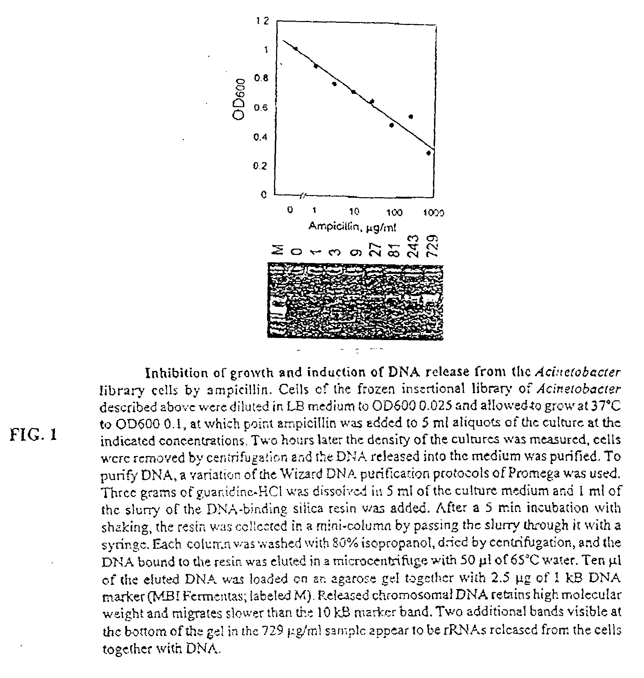

Clones hypersusceptible to the β-lactam drug ampicillin, an antibiotic that is well known to cause bacterial lysis, were selected. As compared to *E*. *coli*, *Acinetobacter* is remarkably resistant to ampicillin. As illustrated in FIG. 1 and Example 1, in liquid culture the growth of *Acinetobacter* was inversely proportional to the logarithm of ampicillin concentration, with a slow growth observed even at 729 µg/ml of the antibiotic. FIG. 1 also shows that the higher the concentration of the antibiotic, the more DNA was released into the medium.

To purify the DNA released, bacterial cells from the insertional library of *Acinetobacter* were diluted in LB medium to OD₆₀₀ 0.025 and allowed to grow at 37 °C to OD₆₀₀ 0.1. Ampicillin was added to 5 ml aliquots of the culture at a variety of concentrations. After two hours, the cells were removed by centrifugation. DNA released into the medium in response to incubation with ampicillin was purified using a variation of the Wizard DNA purification protocols of Promega. Specifically, three grams of guanidine-HCl was dissolved in 5 ml of the culture medium and 1 ml of the slurry of DNA-binding silica resin (Promega Wizard DNA purification resin) was added. After a 5 min incubation with shaking, the resin was collected in a mini-column by passing the slurry through it with a syringe. Each column was washed with 80% isopropanol, dried by centrifugation, and the DNA bound to the resin was eluted in a microcentrifuge with 50 µl of 65°C water. Additional details of the procedure are provided in Example 1.

On the basis of the evidence disclosed in Example 1 and FIG. 1, SDR selection was performed at four concentrations of the antibiotic: 0 (control), 1, 3 and 9 µg/ml, the concentrations which cause only moderate growth inhibition and induce little lysis. The released DNA collected from the corresponding cultures was used to transform fresh *Acinetobacter*: 10 µl (one fifth) of the collected DNA was added to 1 ml of exponentially growing *Acinetobacter* (LB, OD₆₀₀ ~ 0.3) and the culture was incubated for one hour, after which time cells were centrifuged and plated on kanamycin plates to select for transformants. Even the small amount of DNA barely visible on the gel shown in FIG. 1 was sufficient to produce approx. 10-50,000 colonies of transformants. The next morning, cells were washed off the plates, diluted in 5 ml of LB to OD₆₀₀ 0.025 and allowed to grow to OD₆₀₀ 0. 1, at which time ampicillin was added at the corresponding concentrations (*i.e.*, cells transformed with DNA released at 1 µg/ml of ampicillin were incubated with 1 µg/ml of ampicillin, cells transformed with DNA released at 3 µg/ml of ampicillin were incubated with 3 µg/ml of ampicillin). Two hours later the cultures were centrifuged and DNA was purified from the medium as described above, thus starting the next round of selection.

The methods disclosed above can be employed by one of ordinary skill in the art in collecting DNA from mutant libraries of other Gram-negative and of Gram-positive bacteria and selecting library clones hypersusceptible to antibiotic agents.

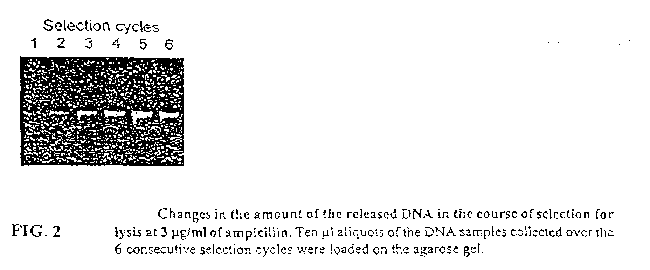

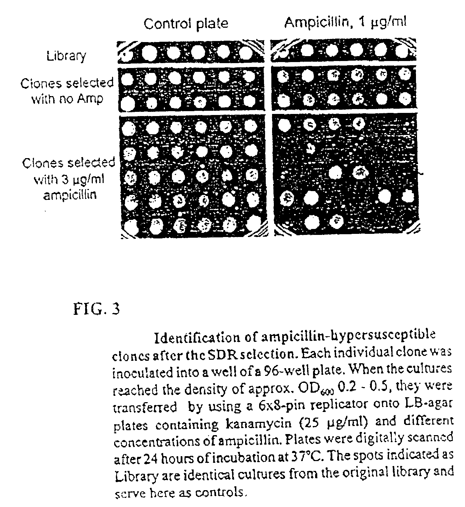

FIG. 2 shows how the amount of released DNA changed over the course of selection. At the beginning of the selection process each selection cycle led to a substantial increase in this amount thus indicating that the selection was indeed happening. However, after the 4^{th} cycle the amount of released DNA remained practically constant. The cells transformed with DNA released during the 5^{th} cycle were subcloned and individual clones were tested for their sensitivity to ampicillin. The typical results of such experiment are presented in FIG. 3.

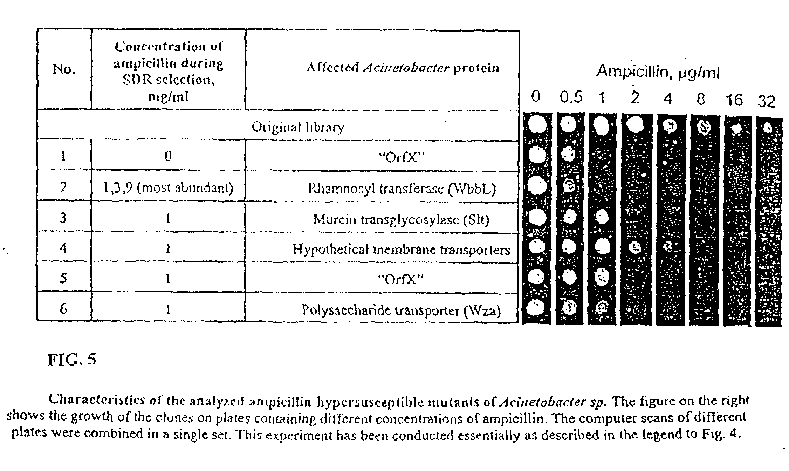

FIG. 3 shows that the SDR selection successfully enriches bacterial population with ampicillin-hypersusceptible clones. While the library cells were growing on plates with as much as 32 µg/ml of ampicillin (FIG. 5), seventeen out of the 30 tested clones selected with 3 µg/ml of ampicillin could not grow in the presence of 1 µg/ml of the antibiotic (FIG. 3). A number of additional clones among these 30 demonstrated less pronounced hypersusceptibility: their growth ceased at 2, 4 or 8 µg/ml of ampicillin. Similar distributions were obtained for clones selected with 1 and 9 µg/ml of ampicillin. The majority of clones obtained after the "selection" with no ampicillin did not demonstrate hypersusceptibility to this antibiotic (FIG. 3). It should be noted, however, that two clones, obtained by such mock selection, were isolated and were as hypersusceptible as the ones selected with ampicillin.

### Non-Lysing Antibiotics Plus Additional Treatments

The present invention encompasses use of non-lysing antibacterial agents under conditions that cause lysis of bacteria. Use of these bacteriostatic antibacterial agents, in combination with additional treatment of the bacteria, results in lysis of bacterial cells affected by the antibacterial agent or combination of agents. Such agents include, but are not limited to macrolide, tetracycline, ketolide, chloramphenicol, lincosamide, oxazolidinone, fluoroquinolone, rifamycin, aminoglycoside, glycopeptide, daptomycin, fusidane, sulphonamide, cycloserine, diaminopyridine, isonicotinic acid or nitrofuran. The additional treatments causing release of the bacterial DNA include, but are not limited to detergents, lysozyme, organic solvents, proteases, chaotropic agents, osmotic shock, mechanical disintegration, or a combination of these treatments.

The SDR method permits selection of DNA released from bacteria lysed in response to treatment with the combination of one or more bacteriostatic antibiotics followed by one or more additional treatments. A library of bacteria, carrying random insertions of a marker gene in their chromosomes, is treated with a bacteriostatic antibacterial agent known in the art at a concentration lower than that normally affecting such bacteria, for instance, a concentration below the minimum inhibitory concentration (MIC). The library of bacteria is then subjected to one or more additional treatments, resulting in lysis of the bacteria and release of bacterial DNA into the medium. For example, a detergent, sodium dodecyl sulfate, can be added to the bacteria at a concentration that lyses cells affected by an antibiotic but not unaffected bacteria. The DNA that is released into the medium is collected in the manner disclosed in the present application. A fresh batch of bacteria is then transformed with the released DNA, using the techniques disclosed herein, and selected for a genetic marker, which integrates into the chromosome of the recipient bacteria. The secondary library thus obtained is subjected to the same treatment as the original library and the cycle is repeated as many times as needed.

### Reporter Strains of Bacteria

If reporter strains of bacteria are employed, the SDR method of the present invention may be used with antibiotics that do not cause bacterial cell lysis. Reporter strains lyse in response to antibiotics that normally do not cause bacterial lysis. The reporter strains of the present invention comprise lysis genes of bacteriophage λ that are placed under control of the bacterial promoters activated by antibiotics. Thus, the disclosed SDR method is applicable to many antibiotics, possibly all of them.

The expression of the lysis cassette of bacteriophage λ in *Acinetobacter* causes lysis of the bacteria and the release of DNA into the medium (Kloos *et al.*, 1994). This 1.3 kB cassette contains three genes: the R and Rz genes that encode phage lysozymes degrading bacterial peptidoglycan, and a preceding S gene that encodes a so-called holin protein. At the final stages of bacteriophage development holin makes pores in the plasma membrane thus allowing R and Rz to escape into the periplasm, where they degrade peptydoglycan and cause bacterial lysis (Young, 1992).

Antibiotics induce the expression of certain bacterial stress-related genes. For example, in *E*. *coli,* chloramphenicol and tetracycline induce the expression of the cold-shock protein gene cspA, whereas aminoglycosides and polymyxin induce heat shock operon *ibp* (Bianchi and Baneyx, 1999). Although there are instances when antibiotic molecules themselves serve as inducers of gene expression (Alekshun and Levy, 1999; Lewis, 1999), in the case of stress genes this induction likely occurs in response to the biochemical effects these antibiotics exert, *e.g.*, inhibition of translation by tetracycline and chloramphenicol or misincorporation of amino acids into proteins for aminoglycosides.

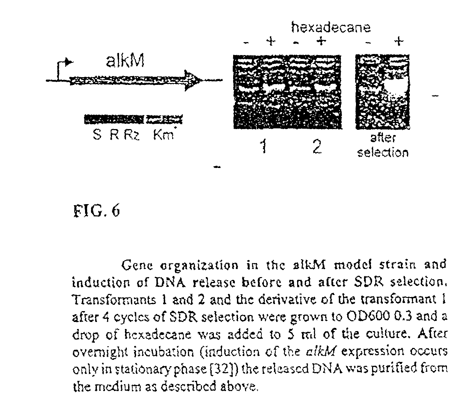

A model strain of *Acinetobacter* with the lysis cassette of λ inserted under the control of a antibiotic-inducible promoter was constructed and determined to release DNA in response to the antibiotic inducer. A promoter of the *alkM* gene of *Acinetobacter*, which encodes an enzyme involved in degradation of hydrocarbons and whose expression is activated dramatically by hexadecane, was chosen (Ratajczak *et al*., 1998). The promoter-less lysis cassette was PCR-amplified from the DNA of λ. The 5'-end of the gene of S-protein, which is the limiting factor in lysis (Young, 1992), was modified by an appropriately designed PCR primer. In particular, the alternative start codon from which an inhibitory variant of S is translated has been eliminated by mutation. This alternative translational start is required to delay lysis until the λ particles are fully developed (Graschopf and Blasi, 1999), but would have only impeded the activity of the lysis cassette. The lysis cassette was attached upstream of the *nptII* gene of transposon Tn5, which provides resistance to kanamycin (FIG. 6). The obtained 2.2 kB construct was cloned into the MIuI site of the *alkM* gene cloned in pUC 18. The resulting construct was then used to transform *Acinetobacter* with selection for kanamycin. Hexadecane caused significant increase in the amount of DNA released from the obtained transformants (FIG. 6), although it had no effect on the release of DNA from the wild-type *Acinetobacter.*

While promoting the release of DNA in the model strain, hexadecane did not cause significant reduction of the optical density of the culture, indicating that lysis was occurring only in a small fraction of cells. The lytic potential of the lysis cassette was increased by performing four rounds of SDR selection with the *alkM* model strain. In each round, the DNA released in the presence of hexadecane was used to transform fresh bacteria. As FIG. 6 illustrates, this selection led to a dramatic enhancement of the lytic response to hexadecane. In fact, cultures of this evolved clone had significantly lower cell density after exposure to hexadecane than controls.

The detailed generation of a lysis reporter strain responding to a translational inhibitor, chloramphenicol, is disclosed in the following Examples. The disclosed methods can be readily adapted by one of skill in the art to generate lysis reporter strains of other Gram-negative and of Gram-positive bacteria.

### Identification of Antibacterial Hypersusceptibility Loci

As noted above, the selection conditions imposed lead to enrichment of the antibiotic hypersusceptibility phenotype in the mutant libraries. The antibacterial hypersusceptibility phenotype co-segregated with the marker, such that identification of the sites of insertion of the marker into a particular genetic locus led to identification of antibacterial hypersusceptibility loci. The site of insertion in each mutant clone was determined by sequencing. Any method of DNA sequencing known in the art may be employed in the present invention. In turn, the gene for a potential target of an antibiotic potentiator was determined.

In order to identify antibacterial hypersusceptibility loci, the sites of insertion of the marker gene in the chromosomes of the mutants in the mutant libraries are determined. DNA isolated from the mutants may be inserted into the vector by a variety of procedures. In general, the DNA sequence is cut with restriction enzymes known in the art and the marker gene, together with the flanking chromosomal sequences, is cloned into a vector, which is then selected for the presence of the marker gene. Confirmation that the vector carries the marker gene is made by transforming bacteria, with selection for the marker. The clones so obtained are then analyzed for the presence of the marker phenotype. The vector is sequenced using techniques well known in the art.

Representative examples of vectors which may be used are viral particles, baculovirus, phage, plasmids, phagemids, cosmids, phosmids, bacterial artificial chromosomes, viral DNA (*e.g.* vaccinia, adenovirus, fowl pox virus, pseudorabies and derivatives of SV40), P1-based artificial chromosomes, yeast plasmids, yeast artificial chromosomes, and any other vectors specific for specific hosts of interest (such as bacillus, aspergillus, yeast, etc.). Thus, for example, the DNA may be included in any one of a variety of vectors for expressing a polypeptide. Such vectors include chromosomal, nonchromosomal and synthetic DNA sequences. Large numbers of suitable vectors are known to those of skill in the art, and are commercially available. The following vectors are provided by way of example; Bacterial: pQE70, pQE60, pQE-9 (Qiagen), psiX174, pBluescript SK, pBluescript KS, pNH8A, pNH16a, pNH18A, pNH46A (Stratagene); pTRC99a, pKK223-3, pKK233-3, pDR540, pRIT5 (Pharmacia); Eukaryotic: pWLNEO, pSV2CAT, pOG44, pXT1, pSG (Stratagene), pSVK3, pBPV, pMSG, pSVL (Pharmacia). However, any other plasmid or vector may be used as long as they are replicable and viable in the host.

In a preferred embodiment, the sites of insertion of the Km^{R} gene in the chromosomes of the selected mutants were identified. DNA isolated from the mutants was cut with restriction enzymes and the Km^{R} gene together with flanking chromosomal sequences was cloned into an *E. coli* plasmid vector and selected for kanamycin resistance. More specifically, the DNA of the mutants was digested with a mixture of four enzymes: XbaI (T'CTAGA), NheI (G'CTAGC, AvrII (C'CTAGG) and BcuI (A'CTAGT). All these enzymes create identical cohesive ends, all work in the same digestion buffer and, most importantly, none of them cuts the KM^{R} cassette used herein. The obtained digest was ligated with the NheI-cut pBR322, a vector capable of carrying large inserts. Kanamycin-resistant plasmid clones were obtained for all of the fifteen analyzed hypersusceptible mutants. To verify that the plasmids carry the insertions of Km^{R} responsible for the hypersusceptibility phenotype of the mutants, these plasmids were used to transform *Acinetobacter* with selection for kanamycin resistance. The clones so obtained were analyzed for their sensitivity to ampicillin in a manner shown in FIG. 3 and in all cases were found to be as hypersusceptible as the mutants from which the plasmids were originated. The plasmids were then sequenced using four primers: two outward oriented primers corresponding to the flanks of the KM^{R} cassette, and two inward oriented primers corresponding to the regions of pBR322 surrounding the NheI site. The sequences obtained were then compared with the sequence of the *Acinetobacter* genome.

One skilled in the art can likely apply the loci identified by the methods above for *Acinetobacter* to other Gram-negative bacterial species. Furthermore, the methods disclosed above can be applied to Gram-positive bacteria by using a transformable species such as *Streptococcus.* One skilled in the art can likely apply the loci identified for the transformable Gram-positive bacteria to other Gram-positive bacteria.

### Generation of an Improved Insertional Library of Acinetobacter.

The disclosed insertional library of *Acinetobacter* used for selection of ampicillin-hypersusceptible mutants had two shortcomings. In many instances, restriction fragments of the *Acinetobacter* chromosome were encountered which had no relation to the actual site of the insertion attached to one or even both sides of the integrated KM^{R} cassette, requiring extended sequencing in order to understand where in the *Acinetobacter* genome the insertions had actually happened. In addition, the library had a strong sequence-specific bias. Sequence analysis showed that recombination events leading to the KM^{R} insertion occurred predominantly at the sites located next to unusually long HpaII+Hin6I, TaqI, or Sau3A1 restriction fragments, meaning that the library was strongly biased toward specific regions of the *Acinetobacter* chromosome with unusually distant location of tetranucleotide restriction sites. Use of four different enzymes only partially alleviated this problem. The presence of this strong bias suggests that many potential hyper-susceptibility mutations may not be represented in the insertional library of *Acinetobacter* used for selection of ampicillin-hypersusceptible mutants.

Preferably, the library for SDR selections has a higher complexity, truly random distribution of insertions and does not contain unnatural combinations of DNA fragments complicating the analysis of mutants.

A library of the fragments of the *Acinetobacter* genome in the *E*. *coli* plasmid vector carrying a marker could be constructed, and *Acinetobacter* transformed with the library of plasmids. The circular plasmids are expected to recombine with the genome in a single Campbell-type act of recombination. However, as has already been mentioned, such recombination is a rare event in *Acinetobacter* (Palmen *et al.*, 1993; Palmen and Hellingwerf, 1997). Furthermore, it has recently been shown that standard ColEI *E*. *coli* plasmid vectors can, to a limited extent, replicate in *Acinetobacter*, thus significantly complicating the system (Gralton *et al.*, 1997).

In a preferred embodiment, DNA of *Acinetobacter* is cut into large fragments using restriction enzymes recognizing hexamicleotide sites. To prevent any sequence bias, several enzymes are used separately *(e.g.,* BamH1, PstI, EcoRI, HindIII). Each set of fragments is cloned into an appropriately cut pZero1 plasmid vector (Invitrogen) which carries zeocin-resistance gene allowing for selection in *E. coli.* Since the multicloning site of this vector is located inside the bacteriotoxic ccdB gene, only the plasmids containing inserts survive in *E. coli.* The libraries obtained with different restriction enzymes are pooled together.

The library plasmids are then randomly cut with DNaseI in the presence of Mn²⁺. The enzyme is used at such a low concentration that an average size plasmid is cut approximately once. Many plasmids are not cut at all and many are cut in the vector region. In neither of these two cases are the ensuing products of these molecules able to recombine with *Acinetobacter* chromosome at the final stage of the procedure. A large portion of the library is cut, however, inside the insert, either once or several times. Plasmids with a single cut eventually produce an insertion of the KM^{R} gene into the *Acinetobacter* chromosome, while plasmids with two or more cuts create a limited size deletion.

In the presence of Mn²⁺, DNase produces double-strand breaks in DNA. The ends cut with DNase are repaired with T4 DNA polymerase and Klenow enzyme and are ligated with phosphorylated oligonucleotide adapters containing, on one side, a blunt end which attaches to the repaired ends of the plasmids and, on the other side, a 3'T overhang, which remains free. The KM^{R} gene is amplified by PCR using Taq polymerase, which is known to attach an extra A nucleotide to the 3' ends of all products. The PCR-amplified KM^{R} and the plasmids containing complementary 3'T overhangs are ligated together and transformed into *E. coli* with selection for kanamycin resistance. These plasmids are then used to transform *Acinetobacter*, where two acts of recombination at the regions flanking the KM^{R} gene lead to the insertion of KM^{R} into the chromosome.

The procedure disclosed immediately above has advantages over the procedure that was used to generate the insertional library of *Acinetobacter* used for selection of ampicillin-hypersusceptible mutants. First, a much more representative library is obtained. The neighboring regions of the *Acinetobacter* DNA are held together by the plasmid vector, therefore the fraction of productively recombining molecules is much higher than in the previously used procedure in which, in order to produce an insertion, the neighboring restriction fragments had to ligate to two flanks of the KM^{R} cassette purely accidentally. Recombination processes are more efficient since the fragments of *Acinetobacter* DNA involved in recombination are on average much longer than in the procedure used before. The goal is to construct a library of approximately 35,000 independent clones (one insertion per 100 bp; ~10 insertions per ORF on average). Such complexity ensures that no potential hypersusceptibility mutations will go undetected.

The second advantage is that no unusual DNA rearrangements occur in the final library, thus simplifying the sequence analysis of selected clones. Since the ligation is based on the so-called TA cloning rather than cloning at the restriction sites, loose fragments of *Acinetobacter* DNA which may potentially arise at the DNase digestion step are not incorporated into plasmids. Finally, since DNase cuts DNA essentially randomly, no sequence-specific bias is present in the final library.

The KM^{R} cassette used in the preferred embodiment is slightly different from the cassette used to generate the insertional library of *Acinetobacter* used for selection of ampicillin-hypersusceptible mutants. Specifically, the rrnB T1 transcriptional terminator used in many *E. coli* expression vectors is attached downstream of the coding region of the *nptI* gene. The presence of the terminator excludes the possibility that an insertion of KM^{R}, which contains strong *trc* promoter, leads to hypersusceptibility by enhancing the expression of downstream genes rather than by disrupting the gene at the site of insertion. Judging by the position of ORFs in the mutants described in FIG. 5, their phenotype cannot be explained by such enhancement, but this remains a possibility for other mutants.

The quality of the library is tested before any selections are performed. To determine how representative and unbiased the library is, a series of PCR reactions are conducted. In these reactions one of the primers is an outward oriented primer in the KM^{R} gene and another primer is represented by one of the several primers to nonessential *Acinetobacter* genes available (from the *alkM* gene, from the rhamnose transglycosylase gene, *etc.).* The total DNA isolated from the library is used as a template. If the library is sufficiently complex and unbiased, in each PCR reaction a set of multiple PCR products is obtained whose lengths correspond to the distances between the genomic primer and the inserted KM^{R} cassettes in the library clones. Significantly different numbers of PCR products obtained for different genomic primers signal the presence of an unacceptable bias in the library.

The ease of analyzing the sites of insertion after the hypersusceptibility mutants are obtained is determined. DNA is isolated from several randomly chosen clones of the library and the sites of KM^{R} insertion are cloned into pBR322 and sequenced. Sequences obtained from the two outward oriented primers of the KM^{R} cassette should correspond to the adjacent or closely located stretches of sequence in the genome of *Acinetobacter.*

### EXAMPLES

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### EXAMPLE 1

### Inhibition of growth and induction of DNA release from the Acinetobacter library cells by ampicillin, with collection and purification of the released DNA.

Cells of a frozen insertional library of *Acinetobacter* were diluted in LB medium to OD₆₀₀ 0.025 and allowed to grow at 37°C to OD₆₀₀ 0.1, at which point ampicillin was added to 5 ml aliquots of the culture at the indicated concentrations. Two hours later the density of the cultures was measured, cells were removed by centrifugation and the DNA released into the medium was purified. To purify DNA, a variation of the Wizard DNA purification protocols of Promega was used. Three grams of guanidine-HCl was dissolved in 5 ml of the culture medium and 1 ml of the slurry of the DNA-binding silica resin was added. After a 5 minute incubation with shaking, the resin was collected in a mini-column by passing the slurry through it with a syringe. Each column was washed with 80% isopropanol, dried by centrifugation, and the DNA bound to the resin was eluted in a microcentrifuge with 50 µl of 65°C water. Ten µl of the eluted DNA was loaded on an agarose gel together with 2.5 µg of 1 kB DNA marker (MBI Fermentas; labeled M) (FIG. 1). Released chromosomal DNA retains high molecular weight and migrates slower than the 10 kB marker band. Two additional bands visible at the bottom of the gel in the 729 µg/ml sample FIG. 1 appear to be rRNAs released from the cells together with DNA.

FIG. 2 shows the changes in the amount of the released DNA in the course of selection for lysis at 3 µg/ml of ampicillin. Ten µl aliquots of the DNA samples collected over the 6 consecutive selection cycles were loaded on the agarose gel.

### EXAMPLE 2

### Identification of ampicillin-hypersusceptible clones after the SDR selection.

Each individual clone was inoculated into a well of a 96-well plate. When the cultures reached the density of approx. OD₆₀₀ 0.2-0.5, they were transferred by using a 6 × 8-pin replicator onto LB-agar plates containing kanamycin (25 µg/ml) and different concentrations of ampicillin. Plates were digitally scanned after 24 hr of incubation at 37°C (FIG.3). The spots indicated as Library are identical cultures from the original library and serve here as controls.

### EXAMPLE 3

### Screening of hypersusceptible colonies for the absence of the most abundant insertion of the Km^{R} cassette.

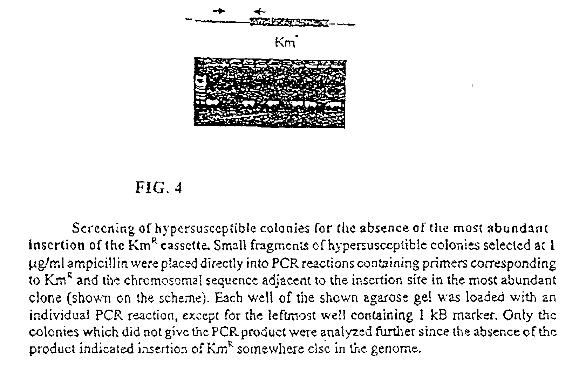

Small fragments of hypersusceptible colonies selected at 1 µg/ml ampicillin were placed directly into PCR reactions containing primers corresponding to KM^{R} and the chromosomal sequence adjacent to the insertion site in the most abundant clone (shown on the scheme) (FIG. 4). Each well of the shown agarose gel was loaded with an individual PCR reaction, except for the leftmost well containing 1 kB marker. Only the colonies which did not give the PCR product were analyzed further since the absence of the product indicated insertion of KM^{R} somewhere else in the genome.

### EXAMPLE 4

### Characteristics of the analyzed ampicillin-hypersusceptible mutants of Acinetobacter sp.

FIG. 5 summarizes the results of sequence analysis for six hypersusceptible clones. Mutants demonstrating the highest level of susceptibility (~32-fold) were analyzed. One randomly chosen mutant with lower level of susceptibility (# 4 in FIG. 5) was also analyzed for comparison.

The figure on the right of FIG. 5 shows the growth of the clones on plates containing different concentrations of ampicillin. The computer scans of different plates were combined in a single set. This study has been conducted essentially as described in Example 2.

Only one mutant, #3, had clear relation to the known mechanism of action of ampicillin. In this mutant, a gene encoding the precursor of murein transglycosylase (homology with the *E*. *coli* protein with this function, Slt, has the BLAST P-score of 1 × 10²³) was completely excised from the chromosome and substituted by the KM^{R} cassette. The Slt protein of *E*. *coli* is a periplasmic murein hydrolase which, together with three other hydrolases, MltA, B and C, is involved in peptidoglycan turnover. The muropeptides produced as a result of the activity of these enzymes serve as inducers of the chromosomal β-lactamase AmpC. It has been shown that the genetic knock-out of Slt has no effect on bacterial growth but significantly reduces the expression of AmpC (Kraft *et al.*, 1999). Furthermore, the known inhibitor of Slt, bulgecin, was shown to increase the sensitivity of *E. coli* to the β-lactam antibiotic cefoxitin.

The other five identified mutations were unexpected. The insertion #4, producing the lowest, 8-fold level of susceptibility, led to the substitution of the KM^{R} cassette for the genes of two putative membrane proteins that are arranged in a single operon. One of these proteins (six putative transmembrane domains) demonstrates moderate homology to the *E*. *coli* homoserine lactone transporter while the other one (two transmembrane domains) has no homologs in the databases.

Four other mutations, #1, 5, 6 and the strongest one, #2, all affect genes involved in the production and transport of extracellular polysaccharides. In the mutant #2, ~30% in the middle of the ORF encoding a close homolog of rhamnosyl transferases from various microorganisms have been substituted by KM^{R}, clearly inactivating the gene. The highest level of homology is with the rhamnosyl transferase of *Serratia marcescens*, WbbL. Rhamnosyl transferase is an enzyme involved in the production of O-antigen, an extracellular polysaccharide attached to LPS. Some mutants of *Serratia marcescens* selected for the resistance to a bacteriophage that uses 0-antigen as a receptor were found to be defective in the production of O-antigen and simultaneously hypersusceptible to ampicillin (Palomar *et al.*, 1995).

Mutant #6 underwent insertion of KM^{R} into the gene whose product is highly homologous to the outer membrane transporters (named Wza in *E. coli*) known to be involved in many bacteria in the export of capsule polysaccharides (Drummelsmith and Whitfield, 2000). Unlike O-antigen, capsule polysaccharides (K antigens in *E. coli*) are not attached to LPS.

Both mutant #1 and 5 had insertions of KM^{R} into the same ORF, although these insertions had different sequence structures and, judging by the sequence, originated from different libraries: mutant from the TaqI library and mutant #5 from the HpaII/Hin6I library. The protein product of this ORF is highly homologous to the so-called OrfX protein encoded in *E*. *coli* next to Wza, where it is a part of the same large operon containing capsule-producing genes. The function of OrfX is unknown since its knock-out produces no detectable effect on capsule biosynthesis (Drummelsmith and Whitfield, 1999). In the genome of *Acinetobacter* the almost indistinguishable OrfX gene (BLAST P-score of 2 × 10⁻⁸²) is encoded apart from Wza and is even localized in a different contig region of the genome sequence. The knock-outs of both OrfX and Wza cause strong hypersusceptibility to ampicillin.

The analysis of ampicillin hypersusceptible mutants obtained by the SDR strategy revealed expected mutations (inactivation of the Slt homolog), but mostly mutations that were entirely unexpected. A number of mutants demonstrating lower levels of hypersusceptibility have also been obtained, but only one of them was analyzed (#4 in FIG. 5).

### EXAMPLE 5

### Generation of the lysis reporter strain responding to translational inhibitors.

A reporter strain, which released DNA in response to chloramphenicol, a bacteriostatic antibiotic which by itself does not cause lysis of bacteria, was created. The most appropriate site of insertion for the lys-KM^{R} cassette was selected using the SDR method. The lysis cassette with an attached KM^{R} gene was amplified by PCR, with primers containing the ClaI sites, from the "evolved" *alkM* model strain. The PCR product was then digested with ClaI and ligated with the fragments of *Acinetobacter* DNA obtained by digestion with either Hin6I or TaqI, restriction enzymes producing cohesive ends compatible with ClaI. *Acinetobacter* was transformed with the ligation mixtures and selected for kanamycin resistance yielding 13,000 (TaqI) and 3,600 (Hin6I) transformants. The libraries were pooled together and the combined library was used for selection.

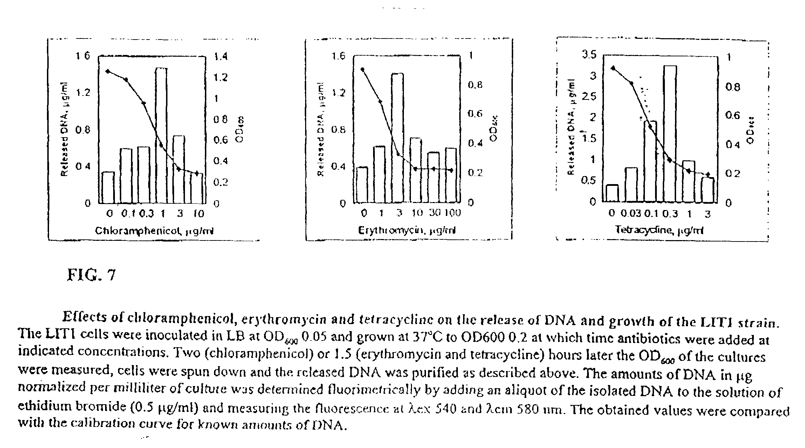

The selection was performed by incubating the exponentially growing library with chloramphenicol, at the concentration of 1.25 µg/ml, for 3 hr and collecting the released DNA that was then used to transform fresh bacteria. Theoretically, insertions downstream of the chloramphenicol-activated promoters had selective advantage over all other insertions. They were passed to the next generation of transformants when chloramphenicol was present in the medium. However, they did not kill cells when chloramphenicol was absent (21 hr a day) and, therefore, during this time multiplied faster than insertions downstream of strong constitutive promoters. After four rounds of selection, the library was subcloned and the effect of chloramphenicol on the release of DNA from individual clones was tested. The inventors were able to identify several clones possessing the desired property. Results for the clone which demonstrated the largest magnitude of response to chloramphenicol are shown in FIG. 7. This clone was designated LIT1 (lysis from inhibitors of translation).

### EXAMPLE 6

### Effects of chloramphenicol, erythromycin and tetracycline on the release of DNA and growth of the LIT1 strain.

The LIT1 cells were inoculated in LB at OD₆₀₀ 0.05 and grown at 37°C to OD600 0.2 at which time antibiotics were added at indicated concentrations. Two (chloramphenicol) or 1.5 (erythromycin and tetracycline) hours later the OD₆₀₀ of the cultures were measured, cells were spun down and the released DNA was purified as described above. The amounts of DNA in µg normalized per milliliter of culture was determined fluorimetrically by adding an aliquot of the isolated DNA to the solution of ethidium bromide (0.5 µg/ml) and measuring the fluorescence at λex 540 and λem 580 nm (FIG. 7). The values obtained were compared with the calibration curve for known amounts of DNA.

The LIT1 clone released four times more DNA in the presence of chloramphenicol than in its absence. Importantly, this clone displayed similar four-fold response to erythromycin and almost ten-fold response to tetracycline. If normalized, not per ml of culture but per number of cells, the amount of released DNA increased in the presence of antibiotics even more significantly: approximately ten-fold in response to chloramphenicol and erythromycin and 35-fold in response to tetracycline. For all three drugs, the highest level of DNA release was observed at antibiotic concentrations producing approximately 80% reduction of the growth rate. At higher concentrations of antibiotics, the amounts of released DNA diminished, probably because the translation of the lysis proteins themselves was inhibited too severely.

### EXAMPLE 7

### Identification of the site of insertion of the lysis gene cassette in the LIT1 strain.

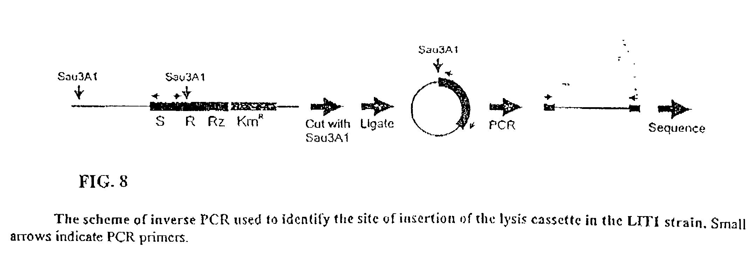

Initially, the same technique was used for identifying the regions of *Acinetobacter* DNA flanking the insertion site as was used for other insertional mutants. However, apparently due to the toxicity of the lysis cassette for *E. coli,* cloning the region of the insertion into pBR322 could not be accomplished. Instead, the inverse PCR approach was used, schematically shown in FIG. 8, which helped to identify the region of *Acinetobacter* DNA directly preceding the lysis cassette. In LIT1, this cassette has inserted upstream of an *ilv* operon whose protein products, acetolactate synthase and ketol-acid reductoisomerase, are involved in the biosynthesis of branched-chain amino acids, isoleucine, leucine and valine. Judging by the location and orientation of the lysis cassette in LIT1, it is under transcriptional control of a surprisingly large (1 kB) regulatory region of this operon, which does not contain any ORFs of significant length.

Unlike *Acinetobacter* which possesses only one *ilv* operon, *E. coli* has three of them, each encoding an isoform of acetolactate synthase. The expression of two of these *ilv* operons is controlled by an attenuation mechanism, in which the expression of an operon is inversely proportional to the rate of translation of a leader ORF containing multiple codons of branched-chain amino acids (Umbarger, 1996). The regulatory region of the *ilv* operon of *Acinetobacter* does not contain sequences that could be interpreted as a leader ORF. Another hallmark of attenuators, an inverted repeat capable of forming a transcriptional terminator, is also absent in this region. Nevertheless, by analogy with *E. coli* and simply from the fact that this operon is involved in the biosynthesis of amino acids, it seems logical that the expression of this operon, might also be controlled in some way by the rate of translation. This would fully explain the induction of lysis of the LIT 1 strain by translational inhibitors.

### EXAMPLE 8

### Selection and analysis of mutations increasing the susceptibility of Acinetobacter to β-lactam antibiotics.

To obtain all the possible mutants hypersusceptible to ampicillin, multiple selections under diverse conditions are conducted and numerous selected clones are analyzed. Varied are the time of treatment with ampicillin (1, 2, or 4 hr), the concentration of ampicillin used for selection (0, 1, 3, 9, 27, 81 µg/ml) and the number of selection cycles (Neyfakh *et al*., 1991; Markham *et al*., 1999; Renau *et al.*, 1999; Vaara, 1993).

In addition to ampicillin, which is a derivative of penicillin, mutants hypersusceptible to a 3^{rd} generation cephalosporin, ceftazidime, and a carbapenem drug, imipenem, two β-lactam antibiotics frequently used to treat *Acinetobacter* infections (Bergogne-Berezin and Towner, 1996) are selected. The spectra of hypersusceptibility mutants is expected to be partially different for these three drugs. The three drugs differ from each other in their affinities for different PBPs (penicillin-binding proteins). In *E. coli,* ampicillin preferentially binds to PBP1a and 1b and causes lysis; ceftazidime preferentially binds to PBP3 and with lower affinity to 1a, thus causing filamentation. and, at higher concentrations, lysis, whereas imipenem binds to PBP2 and 1b causing spheroplast formation and lysis (Neu, 1985; van Langevelde *et al*., 1998). The drugs also differ significantly in their susceptibility to β-lactamases (Kitano and Tomasz, 1979).

To start the selections with ceftazidime and imipenem, a preliminary study, similar to the one shown in FIG. 1, is conducted, in which the concentration dependence of bacterial growth and DNA release for these two antibiotics is determined. After choosing appropriate concentrations for selection (the amount of released DNA exceeding the amount of DNA released in control by 2 - 3 fold), several rounds of selection are performed until the amount of DNA being released reaches a plateau.

The libraries obtained at the end of each selection are subcloned and individual clones are tested for their sensitivity to the β-lactam antibiotics used for selection. These tests are conducted by replica-plating colonies on LB-agar plates with different antibiotic concentrations (see FIG. 5). Numerous clones are tested. The levels of hypersusceptibility are eventually determined for hundreds of clones. The major effort is directed towards characterization of ampicillin-hypersusceptible mutants obtained in the SDR selections with different parameters (time of incubation with ampicillin, its concentration, and the number of rounds of selection). Smaller number of clones (approximately a hundred each) are analyzed for the ceftazidime and imipenem SDR selections since here the goal is limited to evaluating the differences in the spectra of hypersusceptibility mutations selected with different β-lactams.

The sites of insertion of the KM^{R} gene is determined essentially in the same way as disclosed above: DNA isolated from mutants is digested with the mixture of four restriction enzymes which do not cut the KM^{R} gene and create identical 5' overhangs: XbaI, NheI, BcuI and AvrII, and cloned into the NheI site of pBR322 with selection for kanamycin resistance. The plasmids are sequenced from two outward oriented primers of the KM^{R} cassette.

The levels of hypersusceptibility is determined for a large number of clones. The insertion sites are sequenced for ~10 mutants from each selected group (e.g., ampicillin- selected clones demonstrating 32 - 64 fold hypersusceptibility). The obtained sequence information is used to order a primer that will help to exclude clones with the most abundant KM^{R} insertion. This is done essentially in the same way it was done for the most abundant clone as disclosed above (FIG. 4). Specifically, PCR of colonies is performed using one primer next to the site of insertion in the most abundant clone and an outward oriented primer within the KM^{R} gene.

Clones containing the most abundant insertion are excluded from further analysis and the next group of ~10 clones, which do not have this insertion, are sequenced. The same procedure is performed with the next most abundant clone. As this reiteration process progresses, the set of "diagnostic" primers located next to the already determined sites of insertion grows. The primers are designed in such a way that they have approximately identical melting temperatures. This allows them to perform very simple "diagnostic" PCR reactions with colonies. Each PCR reaction contains an outward primer of the KM^{R} cassette and a mixture of diagnostic primers. Only the clones which do not produce a PCR product in any of the reaction are subjected to sequencing.

At the end of this process nearly all of the β-lactam hypersusceptibility mutations that can possibly occur in *Acinetobacter* are identified. Many of the obtained mutations readily suggest the molecular mechanism by which they exert the hypersusceptibility effect, *e.g.,* disruption of the β-lactamase gene. Of most interest are the mutations whose relation to β-lactams are not immediately clear. Ultimately, the proteins affected by these mutations may prove to be the targets of choice for β-lactam potentiators of the future.

### EXAMPLE 9

### Biochemical consequences of the polysaccharide mutations in Acinetobacter.

In the mutants obtained the KM^{R} cassette either inserted into an affected gene or produced a deletion of a significant portion of a gene, while leaving the upstream portion of the gene intact. It is desirable to obtain genetically "clean" knock-outs of the genes *wbbL*, *wza* and *orfX* and verify that inactivation of these genes causes the hypersusceptibility phenotype. This is done by producing precise deletions (from the start codon to the stop codon) of each of these genes. To achieve this, the KM^{R} cassette is ligated on both sides with ~750 bp DNA fragments flanking the gene and the resulting construct is used to transform *Acinetobacter* with selection for kanamycin resistance. Afterwards, the inserted KM^{R} cassette is precisely deleted by transforming the kanamycin-resistant clones with a PCR product in which the flanking regions of the chromosome are joined together, and kanamycin-sensitive clones are identified. This last step, practically impossible in most other bacteria, is easily attainable in *Acinetobacter* with its extremely high rate of transformation. Indeed, the efficiency of transformation in this species is such that ~10% of cells integrate into their chromosome a fragment of DNA with large regions of homology if it is simply added into the medium (Palmen *et al*., 1993). Simple replica plating of a few dozen clones on kanamycin and control plates should identify clones that lost the KM^{R} cassette. All of the DNA manipulations involved were performed by PCT as shown in FIG. 9 which provides additional details.

The deletion clones obtained are tested for susceptibility to ampicillin as shown in FIG. 5. Assuming that they demonstrate the hypersusceptibility phenotype, the inventors characterize changes in polysaccharide production, which occurred in these clones. Judging by the very strong homologies, the WbbL protein is involved in the biosynthesis of O-antigen, a polysaccharide attached to LPS, whereas Wza is an outer membrane protein implicated in the export of precursors for the capsule polysaccharides that are not attached to the LPS. The function of the OrfX homologs is not known, although in *E. coli* and *Klebsiella pneumoniae* (but not in *Acinetobacter)* this protein is encoded within the capsule-producing cluster of genes next to Wza.

Preliminary studies with the original *wbbL* knock-out mutant obtained by the SDR selection (#2 in FIG. 5) demonstrated that LPS in this mutant is devoid of the attached O-antigen (FIG. 10).

Similar electrophoresis studies are performed with the "clean" knock-out mutants of all three genes. Additionally, the production of capsule polysaccharide is analyzed in all three mutants. A modification of the silver staining procedure in which the SDS-PAGE gel is first treated with a solution of a polysaccharide-binding dye, alician blue, is used to detect capsule polysaccharides (Pelkonen and Finne, 1989; Corzo *et al.*, 1991). These studies demonstrate whether the functions of WbbL and Wza in *Acinetobacter* are constrained, like in other Gram-negative bacteria, to O-antigen and capsule polysaccharide respectively, or they both produce similar effects on molecules of both types. The latter possibility is suggested by the fact that the colonies of the original mutants (#2 and 6 in FIG. 5) look distinct from those of wild-type bacteria (more transparent, as it is clearly seen in FIG. 5) and indistinguishable from each other. Since the morphology of the mutant cells does not differ from that of wild type cells, at least under phase contrast examination, this difference in the colony appearance is likely to reflect the difference in the production of extracellular material. The mutants of OrfX (#1 and 5 in FIG. 5) have very similar colony appearance, suggesting that this protein, which reportedly plays no detectable role in the capsule production in *E. coli* (Drummelsmith and Whitfield, 1999) is in fact involved in exopolysaccharide production in *Acinetobacter*.

### EXAMPLE 10

### Generation of the lysis reporter strain responding to fluoroquinolones and selection of fluoroquinolone-hypersusceptible mutants.

Fluoroquinolones are known to induce global stress response in bacteria. Both "old" and "new" quinolones, including the currently most frequently prescribed ciprofloxacin, have been shown to induce the SOS response with the expression of a number of DNA repair genes being activated (Dalhoff and Doring, 1987; Phillips *et al.*, 1987; Ysern *et al.*, 1990). This induction is not surprising since fluoroquinolones act by associating with topoisomerases and inhibiting the relegation of cleaved DNA strands, thus producing DNA breaks which induce the SOS system..

The approach, similar to that used with the LIT1 strain, is to select the most suitable site of insertion of the lyt-KM^{R} cassette by the SDR strategy rather than trying to guess the most optimal site of insertion from the list of known genes of the SOS regulon. The lysis cassette proved to be too toxic for *E. coli* when cloned into pBR322, therefore the "old" library which was originally used to obtain the LIT1 strain is used. This library is selected by the SDR method for the ability to lyse in the presence of ciprofloxacin. The highest level of induction of SOS regulon is observed at the minimal inhibitory concentration of fluoroquinolones (Sullivan *et al*., 1976), thus this concentration is used to promote DNA release during the selection. Specifically, cells of the library are subjected to the presence of ciprofloxacin for two hours, the DNA released into the medium is collected and used to transform fresh *Acinetobacter* with selection for kanamycin resistance. The transformants are allowed to grow for a day to eliminate the cells with constitutively high expression of the lysis cassette, after which time the next round of selection with ciprofloxacin begins. After several rounds, the library is subcloned and individual clones are tested for the induction of DNA release in response to ciprofloxacin.

The chosen clone is further selected by the SDR method for regulatory mutants in which ciprofloxacin produces the strongest response and the obtained derivative is analyzed by sequencing the regions flanking the lys-KM^{R} cassette. The inverse PCR approach (FIG. 8) is used is the site of insertion into pBR322 cannot be recloned.

The KM^{R} gene is deleted from the obtained lysis reporter strain, the library of KM^{R} insertions is transformed into the strain obtained and the SDR selection with ciprofloxacin at the concentrations below the MIC is initiated. After several rounds of selection, the released DNA is transformed into the wild-type *Acinetobacter*, and the clones hypersusceptible to ciprofloxacin are identified by plating on plates with different concentrations of the drug. The sites of insertions are identified by sequencing.

The SDR method, especially when combined with the concept of a lysis reporter strain, can potentially be applicable to solving various biological problems. Indeed, unlike any other method, it allows for the direct selection of sequences producing modified, or even entirely novel regulatory responses. The more practically useful application of the SDR method is to identify bacterial mutations providing hypersusceptibility to antibacterial agents that are not analyzed in the present disclosure, including scores of antibiotics which in the early days of antibiotic research were found to be insufficiently effective for antibacterial therapy. Considering the rising incidence of antibiotic resistance among bacterial pathogens, the "strong" antibiotics of today may eventually have to be replaced with "weak" antibiotics enforced with their potentiators. The SDR method can help identify such potentiators.

All of the methods disclosed and claimed herein can be made and executed without undue experimentation in light of the present disclosure. While the compositions and methods of this invention have been described in terms of preferred embodiments, it will be apparent to those of skill in the art that variations may be applied to the methods and in the steps or in the sequence of steps of the method described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

### REFERENCES

The following references, to the extent that they provide exemplary procedural or other details supplementary to those set forth herein, are specifically incorporated herein by reference.
Ahmed, Lyass, Markham, Taylor, Vazquez-Laslop, Neyfakh, "Two highly similar multidrug transporters of *Bacillus subtilis* whose expression is differentially regulated," *J Bacteriol.*, 1995; 177(14): 3904-3910.
Alekshun and Levy, "The mar regulon: multiple resistance to antibiotics and other toxic chemicals," *Trends Microbiol.,* 1999, 7(10):410-413.
Bergogne-Berezin E, Towner KJ. Acinetobacter spp. as nosocomial pathogens: microbiological, clinical, and epidemiological features. *Clin Microbiol Rev.* 1996, 9(2):148-65.
Bernadsky, Beveridge, Clarke, "Analysis of the sodium dodecyl sulfate-stable peptidoglycan autolysins of select gram-negative pathogens by using renaturing polyacrilamide gel electrophoresis," *J Bacteriol*., 1994, 176(17)5225-5232.
Bianchi and Baneyx, "Stress responses as a tool to detect and characterize the mode of action of antibacterial agents," *Appl Environ Microbiol.,* 1999, 65(11):5023-5027.
Blechschmidt, Borneleit, Kleber, "Purification and characterization of an extracellular beta-lactamase produced by Acinetobacter calcoaceticu," *J Gen Microbiol.*, 1992, 138( Pt 6):1197-1202.
Corzo, Perez-Galdona, Leon-Barrios, Gutierrez-Navarro, "Alcian blue fixation allows silver staining of the isolated polysaccharide component of bacterial lipopolysaccharides in polyacrylarnide gels," *Electrophoresis,* 1991, 12(6):439-441.
Dalhoff and Doring, "Action of quinolones on gene expression and bacterial membranes," *Aritiblot Chemother.,* 1987, 39:205-214.
Drummelsmith and Whitfield, "Gene products required for surface expression of the capsular form of the group I K antigen in *E. coli* (09a:K30)," *Mol Microbiol.,* 1999, 31(5):1321-1332.
Drummelsmith and Whitfield," "Translocation of group I capsular polysaccharide to the surface of *E. coli* requires a multimeric complex in the outer membrane," *EMBO J.,* 2000, 19(1):57-66.
Gill, Brenwald, Wise, "Identification of an efflux pump gene, pmrA, associated with fluoroquinolone resistance in Streptococcus pneumoniae," *Antimicrob Agents Chemother*, 1999; 43(1):187-189.
Goryshin, Jendrisak, Hoffman, Meis, Reznikoff, "Insertional transposon mutagenesis by electroporation of released Tn5 transposition complexes," *Nat Biotechnol.*, 2000, 18(1):97-100.
Gralton, Campbell, Neidle, "Directed introduction of DNA cleavage sites to produce a high-resolution genetic and physical map of the Acinetobacter sp. strain ADP I (BD413UE) chromosome," *Microbiology,* 1997, 143 (Pt 4):1345-1357.
Graschopf and Blasi, "Molecular function of the dual-start motif in the lambda S holin," *Mol Microbiol*., 1999, 33(3):569-582.
Hsieh, Siegel, Rogers, Davis, Lewis, "Bacteria lacking a multidrug pump: a sensitive toot for drug discovery," *Proc Natl Acad Sci USA*, 1998 Jun 9, 95(12):6602-6606.
Hurley, "Beta-lactam antibiotic-induced release of free lipopolysaccharide," *J Infect Dis.,* 1993, 167(3): 775-777.
Jacobs, Huang, Bartowsky, Normark, Park, "Bacterial cell wall recycling provides cytosolic muropeptides as effectors for beta-lactamase induction," *EMBO J.,* 1994, 13(19):4684-4694.
Juni and Janik, "Transformation of Acinetobacter calco-aceticus (*Bacterium anitratum*)," *J Bacteriol*., 1969; 98(1):281-288.
Kitano and Tomasz, "Triggering of autolytic cell wall degradation in *E. coli* by beta-lactam antibiotics," *Antimicrob Agents Chernother.,* 1979, 16(6):838-848.
Kloos, Stratz, Guttler, Steffan, Timmis, "Inducible cell lysis system for the study of natural transformation and environmental fate of DNA released by cell death," *J Bacteriol.*, 1994, 176(23):7352-7361.
Kraft, Prabhu, Ursinus, HoltJe, "Interference with murein turnover has no effect on growth but reduces beta-lactamase induction in *Escherichia coli*," *J Bacteriol.*, 1999, 181(23):7192-7198.
Kraft, Templin, Holtje, "Membrane-bound lytic endotransglycosylase in *E. coli,*" *J Bacteriol*., 1998, 180(13):3441-3447.
Levy and Nelson, "Reversing tetracycline resistance. A renaissance for the tetracycline family of antibiotics," *Adv Exp Med Biol.*, 1998, 456:17-25.
Lewis, "Multidrug resistance: Versatile drug sensors of bacterial cells," *Curr Biol.,* 1999, 9(1 1):R403-407.
Li, Ma, Livermore, Nikaido, "Role of efflux pump(s) in intrinsic resistance of Pseudomonas aeruginosa: active efflux as a contributing factor to beta-lactam resistance," *Antimicrob Agents Chemother.,* 1994, 38(8):1742-1752.
Markham, Westhaus, Klyachko, Johnson, Neyfakh, "Multiple novel inhibitors of the NorA multidrug transporter of *Staphylococcus aureus*, *Antimicrob Agents Chemother.,* 1999, 43(10):2404-2408.
McMurry and Levy, "Tn5 insertion in the polyriboucleotide phosphorylase (pnp) gene in *E. coli* increases susceptibility to antibiotics," *J Bacteriol.*, 1987; 169(3):1321-1324.
Mitscher, "Antibiotics and antimicrobial agents," *In: Principles of Medicinal Chemistry,* 4th Edition (Foye *et al.*, eds.), 1995, pp. 759-801.
Morrison, "Streptococcal competence for genetic transformation: regulation by peptide pheromones," *Microb Drug Resist.,* 1997 3(1):27-37.
Nelson and Levy, "Reversal of tetracycline resistance mediated by different bacterial tetracycline resistance determinants by an inhibitor of the Tet(B) antiport protein," *Antimicrob Agents Chemother,* 1999, 43(7):1719-1724.
Neu, "Relation of structural properties of beta-lactam antibiotics to antibacterial activity," *Am J Med.,* 1985, 79(Suppl. 2A):2-13.
Neyfakh, Bidnenko,, Chen, "Efflux-mediated multidrug resistance in *Bacillus subtilis*: similarities and dissimilarities with the mammalian system," *Proc Natl Acad Sci USA*, 1991, 88(11):4781-4785.
Niga, Yoshida, Hattori, Nakamura, Ito, "Cloning and sequencing of a novel gene (recG) that affects the quinolone susceptibility of *Staphylococcus aureus,*" *Antimicrob Agents Chemother.,* 1997, 41(8):1770-1774.
Nikaido, "Multiple antibiotic resistance and efflux," *Curr Opin Microbiol.,* 1998, 1(5):516-523.
Novak, Charpentler, Braun, Tuomanen, "Signal transduction by a death signal peptide: uncovering the mechanism of bacterial killing by penicillin," *Mol Cell,* 2000;5(1):49-57.
Onishi, Pelak, Gerckens, Silver, Kahan, Chen, Patchett, Galloway, Hyland, Anderson, Raetz, "Antibacterial agents that inhibit lipid A biosynthesis," *Science,* 1996;274(5289): 980-982.
Palmen and Hellingwerf, "Uptake and processing of DNA by Acinetobacter calcoaceticus review," *Gene,* 1997, 192(1):179-190.
Palmen, Vosman, Buijsman, Breek, Hellingwerf, "Physiological characterization of natural transformation in Acinetobacter calcoaceticus. *J Gen Microbiol.* 1993, 139(Pt 2):295-305.
Palmen, Vosman, Kok, van der Zee, Hellingwerf, "Characterization of transformation- deficient mutants of Acinetobacter calcoaceticus," *Mol Microbiol.,* 1992, 6(13):1747-1754.
Palomar, Puig, Montilla, Loren, Vinas, "Lipopolysaccharide recovery restores susceptibility levels towards beta-lactams in Serratia marcescens," *Microbios* 1995, 82(330):21-26.
Paul, Joly-Guillou, Bergogne-Berezin, Nevot, Philippon, "Novel carbenicillin-hydrolyzing beta-lactamase (CARB-5) from Acinetobacter calcoaceticus var. anitratus," *FEMS Microbiol Lett.,* 1989, 50(1-2):45-50.
Pelkonen and Finne, "Polyacrylamide gel electrophoresis of capsular polysaccharides of bacteria," *Methods Enzymol*., 1989 179:104-110.
Phillips, Culebras, Moreno, Baquero, "Induction of the SOS response by new 4-quinolones," *J Antimicrob Chemother.,* 1987, 20(5):631-638.
Potvin, Leclerc, Tremblay, Asselin, Bellemare, "Cloning, sequencing and expression of a Bacillus bacteriolytic enzyme in *E. coli*," *Mol Gen Genet.,* 1988, 21:241-248.
Powell and Young, "Lysis of *E*. *coli* by beta-lactams which bind penicillin-binding proteins I a and I b: inhibition by heat shock proteins," *J Bacteriol*., 1991, 173(13):4021-4026.
Ratajczak, Geissdorfer, Hillen, "Expression of alkane hydroxylase from Acinetobacter sp. strain ADPI is induced by a broad range of n-alkanes and requires the transcriptional activator AlkR, *J Bacteriol.*, 1998, 180(22):5822-5827.
Renau, Leger, Flamme, Sangalang, She, Yen, Gannon, Griffith, Chamberland, Lomovskaya, Hecker, Lee, Ohta, Nakayama, "Inhibitors of efflux pumps in Pseudomonas aeruginosa potentiate the activity of the fluoroquinolone antibacterial levofioxacin," *J Med Chem.,* 1999; 42(24):4928-4931.
Rodionov and Ishiguro, "Direct correlation between overproduction of guanosine 3',5'-bispyrophosphate (ppGpp) and penicillin tolerance in *E*. *coli,*" *J Bacteriol.*, 1995, 177(15): 4224-4229.
Rodionov, Pisabarro, de Pedro, Kusser, Ishiguro, "Beta-lactam-induced bacteriolysis of amino acid-deprived Escherichia coli is dependent on phospholipid synthesis," *J Bacteriol.*, 1995, 177(4992-4997.
Saint, Alexander, McClure, pTIM3, a plasmid delivery vector for a transposon-based inducible marker gene system in gram-negative bacteria. *Plasmid*, 1995, 34(3):165-74.
Schiffer and Holtje, "Cloning and characterization of PBP I C, a third member of the multimodular class A penicillin-binding proteins of *E. coli,*" *J Biol Chem.,* 1999, 274(45):32031-32039.
Sullivan, Valois, Watson, *In: Mechanisms in Bacterial Toxicology* (Bernheinerm, ed.) 1976, p. 217, Wiley, NY.
Tsai and Frasch, "A sensitive silver stain for detecting lipopolysaccharides in polyacrylamide gels," *Anal Biochem.*, 1982, 119:115-119.
Umbarger, "Biosynthesis of branched-chain amino acids," *In: Escherichia coli and Salmonella,* Neidhardt (ed.), 1996, pp. 442-457, ASM Press, Washington DC.
Vaara, "Antibiotic-supersusceptible mutants of *E*. *coli* and *Salmonella typhimurium,*" *Antimicrob Agents Chemother.,* 1993; 37(11):2255-2260.
van Langevelde, Kwappenberg, Groeneveld, Mattie, van Dissel, "Antibiotic-induced lipopolysaccharide (LPS) release from Salmonella typhi: delay between killing by ceftazidime and imipenem and release of LPS," *Antimicrob Agents Chemother.,* 1998;42(4):739-743.
Wang and Bechhofer, "Properties of a *Bacillus subtilis* polynucleotide phosphorylase deletion strain," *J Bacteriol*, 1996; 178(8):2375-2382.
Westphal and Jann, "Bacterial Lipopolysaccharides: Extraction with phenol-water and further applications of the procedure," *In: Methods in Carbohydrate Chemistry,* Whistler (ed.), 1965, 5:83-92, Academic Press, NY.
Yamada, Kurose-Hamada, Fukuda, Mitsuyama, Takahata, Minami, Watanabe, Narita, "Quinolone susceptibility of norA-disrupted Staphylococcus aureus," *Antimicrob Agents Chemother.*, 1997; 41(10):2308-2309.
Young, "Bacteriophage lysis mechanism and regulation," *Microbiol Rev.,* 1992, 56(3):430-481.
Ysern, Clerch, Castano, Gibert, Barbe, Llagostera, "Induction of SOS genes in *E. coli* and mutagenesis in Salmonella typhimurium by fluoroquinolones, *Mutagenesis*, 1990, 5(1):63-66.

## Claims

1. A method for identifying an antibacterial hypersusceptibility locus comprising:
a) mutagenizing a bacterial population by random insertional mutagenesis, wherein said mutagenesis results in the insertion of a selectable or screenable marker into the DNA of bacteria in said population;
b) treating the mutagenized bacterial population with an antibacterial agent or a combination of agents at a concentration below that normally affecting said bacteria;
c) collecting DNA from the mutant bacterial cells affected by said treatment; and
d) determining the location of said selectable or screenable marker in DNA of step (c),
wherein said location of said selectable or screenable marker identifies said antibacterial hypersusceptibility locus.

2. The method of claim 1, in which the location of said susceptibility locus is determined by transforming a second bacterial population with the collected DNA of step (c), selecting transformants for the integration of said selectable marker, and determining the location of said selectable marker in DNA isolated from the transformants of step (a).

3. The method of claim 1, further comprising, before the determining step, transforming a second bacterial population with the collected DNA of step (c), selecting transformants for the integration of said selectable marker, treating the population of transformants with the antibacterial agent or a combination of agents, and collecting DNA from the bacterial cells affected by said treatment.

4. The method of claim 3, further comprising an additional round of transformation, agent treatment and DNA collection prior to determining the location of said locus.

5. The method of claim 3, further comprising multiple additional rounds of transformation, antibacterial treatment and DNA collection prior to determining the location of said locus.

6. The method of claim 1, wherein said antibacterial agent or combination of agents cause bacterial lysis.

7. The method of claim 6, wherein antibacterial agent or combination of agents comprises a β-lactam antibiotic.

8. The method of claim 7, wherein said β-lactam antibiotic is penicillin, mecillinam, cephalosporin, clavam, 1-oxacephem, 1-carbapenem, olivanic acid, thienamycin, imipenem, nocardicin or momobactam.

9. The method of claim 6, wherein at least one of said antibacterial agents comprises a disinfectant.

10. The method of claim 6, wherein at least one of said antibacterial agents comprises human blood or blood component causing bacterial lysis.

11. The method of claim 10, wherein said blood or blood component is plasma, serum, antibacterial antibodies, purified components of the complement system, or their combinations.

12. The method of claim 1, wherein said antibacterial agent or combination of agents do not cause bacterial lysis, and said bacterial populations are genetically modified to undergo lysis in response to said agents.

13. The method of claim 12, wherein each member of said bacterial populations contains a lysis gene cassette under the transcriptional control of a promoter activated by said antibacterial agents.

14. The method of claim 12, wherein said lysis gene cassette is a bacteriophage lysis gene.

15. The method of claim 13, wherein said lysis gene cassette is inserted into the bacterial chromosome under the control of a chromosomal promoter.

16. The method of claim 13, wherein said lysis gene cassette is inserted into an extrachromosomal vector.

17. The method of claim 6, wherein DNA is collected from the culture medium.

18. The method of claim 17, wherein collection is by absorption on silica resin in the presence of guanidine, followed by elution with a water solution of low ionic strength.

19. The method of claim 1, wherein DNA is collected following treatment with additional agents, wherein bacterial cells affected by said antibacterial agent or combination of agents release DNA upon the additional treatments, and bacterial cells not affected by said antibacterial agent or combination of agents do not release DNA upon the additional treatments.

20. The method of claim 19, wherein said additional treatments include detergents, lysozyme, organic solvents, proteases, chaotropic agents, osmotic shock, mechanical disintegration, or combinations of these treatments.

21. The method of claim 1, wherein DNA is collected following separation of bacterial cells affected by said antibacterial agent or combination of agents from bacterial cells not affected by said antibacterial agent or combination of agents.

22. The method of claim 21, wherein separation of affected bacterial cells from unaffected bacterial cells is performed on the basis of gene expression, motility, morphology, buoyant density, absorption properties, affinity for antibodies, optical properties, fluorescence properties, ability to produce fluorescent metabolites, or a combination thereof.

23. The method of claim 12, wherein said antibacterial agent or combination of agents comprises an antibiotic.

24. The method of claim 23, wherein said antibiotic is a macrolide, tetracycline, ketolide, chloramphenicol, lincosamide, oxazolidinone, fluoroquinolone, rifamycin, aminoglycoside, glycopeptide, daptomycin, fusidane, sulphonamide, cycloserine, diaminopyridine, isonicotinic acid or nitrofuran.

25. The method of claim 12, wherein said antibacterial agent or combination of agents comprises a disinfectant.

26. The method of claim 25, wherein said disinfectant is an alcohol, aldehyde, anilide, biguanide, diamidine, halogen-releasing agent, silver compound, peroxygen compound, phenol, bis-phenol, halophenol or quaternary ammonium compound.

27. The method of claim 1, wherein said bacterial population *is Acinetobacter sp.*, *Haemophillus influenzae, Mycobacterium spp., Neisseria gonorrheae, Streptococcus spp., Micrococcus spp., Lactococcus spp., Corynebacterium spp., Staphylococcus spp. Pseudomonas aeruginosa, Enterococcus spp., Escherichia coli, Bacillus spp., Enterobacter spp.,* *Citrobacter spp*., *Serratia spp., Listeria spp., Proteus spp.*, *Salmonella spp., Klebsiella spp., Shigella spp*., *Chlamydia spp.*, *Coxiella spp., Bordetlla spp., Legionella pneumophilia, Virbrio cholera*, *Treponema pallidum*, *Rickettsia spp., Borrela spp. Campylobacter spp., Yersinia spp. and Helicobacter pylori.*

28. The method of claim 1, wherein said marker is a screenable marker.

29. The method of claim 28, wherein said screenable marker comprises a gene encoding an enzyme producing a colored product, a gene encoding an enzyme producing a fluorescent product, or a fragment of DNA that can be detected by molecular hybridization or polymerase chain reaction.

30. The method of claim 1, wherein said marker is a selectable marker.

31. The method of claim 30, wherein said selectable marker comprises an antibiotic-resistance gene, metal-resistance gene, nutrient-utilization gene, or a gene encoding an enzyme compensating for a mutation in said bacteria.

32. The method of claim 1, wherein mutagenizing comprises a transposon insertion, a prophage insertion, an integron insertion, or an insertion by homologous recombination.

33. The method of claim 1, wherein the antibacterial hypersusceptibility locus comprises a gene or operon.

34. The method of claim 33, further comprising isolating a full length copy of said gene or operon.

35. The method of claim 34, further sequencing said gene or operon.

36. The method of claim 33, further comprising identifying said gene or operon from a sequenced bacterial genome.
